# FASCICULE DE BREVET EUROPEEN

(11) **EP 1 791 872 B1**
(45) Date de publication et mention de la délivrance du brevet: **07.01.2009**
(21) Numéro de dépôt: 05805571.6
(22) Date de dépôt: 07.09.2005
(51) Int. Cl.: C08B 37/00, A61K 31/715

(54) **HEXADECASACCHARIDES BIOTINYLES, COMPOSITIONS PHARMACEUTIQUES ET LEUR UTILISATION**
BIOTINYLIERTE HEXADECASACCHARIDE, PHARMAZEUTISCHE ZUSAMMENSETZUNGEN DARAUS UND DEREN VERWENDUNG
BIOTINYLATED HEXADECASACCHARIDES, PHARMACEUTICAL COMPOSITIONS AND USE THEREOF

(30) Priorité: 09.09.2004 FR 0409557
(43) Date de publication de la demande: 06.06.2007
(73) Titulaire: Sanofi-Aventis, 75013 Paris (FR)
(72) Inventeur: DUCHAUSSOY, Philippe, F-31200 Toulouse (FR); HERAULT, Jean, Pascal, F-31270 Frouzins (FR); HERBERT, Jean, Marc, F-31170 Tournefeuille (FR); PETITOU, Maurice, F-75645 Paris Cedex 13 (FR); SAVI, Pierre, F-31600 Seysses (FR)
(74) Mandataire: Morel-Pécheux, Muriel
(86) Numéro de dépôt international: PCT/FR2005/002218
(87) Numéro de publication internationale: WO 2006/030104

(56) Documents cités:
- WO-A-02/24754

## Description

La présente invention concerne de nouveaux hexadécasaccharides biotinylés de synthèse possédant les activités pharmacologiques anticoagulante et antithrombotique de l'héparine.

L'héparine catalyse, notamment via l'antithrombine III (AT III), l'inhibition de deux enzymes qui interviennent dans la cascade de la coagulation du sang, à savoir, le facteur Xa et le facteur IIa (ou thrombine). Les préparations d'héparines de bas poids moléculaire (HBPM), contiennent des chaînes formées de 4 à 30 monosaccharides et ont la propriété d'agir plus sélectivement sur le facteur Xa que sur la thrombine.

Il est connu que l'inhibition du facteur Xa nécessite une fixation de l'héparine sur l'AT III via le domaine de liaison à l'antithrombine (Domaine-A), et que l'inhibition du facteur IIa (thrombine) nécessite une fixation à l'AT III, via le Domaine-A, ainsi qu'à la thrombine via un domaine de liaison moins bien défini (Domaine-T).

Les oligosaccharides de synthèse correspondant au domaine Domaine-A de l'héparine sont connus. Ils sont décrits par exemple dans les brevets EP 84999 et EP 529715, la demande de brevet publiée sous le numéro WO 99/36428 et la publication Bioorg. Med. Chem. (1998), 6, pp.1509-1516. Ces oligosaccharides de synthèse ont la propriété d'inhiber sélectivement, via l'AT III, le facteur Xa de la coagulation sans aucune activité sur la thrombine. Ils manifestent une activité antithrombotique dans la thrombose veineuse.

Des oligosaccharides de synthèse capables d'inhiber la thrombine et le facteur Xa via l'activation de l'AT III ont été décrits dans les demandes de brevet publiées sous les numéros WO 98/03554 et WO 99/36443.

Dans ces demandes de brevet sont décrits de nouveaux dérivés polysaccharidiques sulfatés et alkylés biologiquement actifs. Ils sont en particulier anticoagulants et antithrombotiques. Il a en particulier été montré que ces polysaccharides sulfatés et alkylés peuvent être de puissants antithrombotiques et des anticoagulants selon la disposition des groupes alkyles et des groupes sulfates portés par le squelette glucidique. De façon plus générale, il a été trouvé que par réalisation de séquences polysaccharidiques il est possible de moduler avec précision les activités de type GAGs pour obtenir des produits très actifs présentant les propriétés pharmacologiques anticoagulantes et antithrombotiques de l'héparine. Par rapport à l'héparine, ils présentent l'avantage d'être d'une structure déterminée et de ne pas réagir avec le facteur 4 plaquettaire, cause des effets thrombocytopéniants de l'héparine.

Cependant, l'usage en thérapeutique humaine de certains produits décrits dans les demandes de brevet publiées sous les numéros WO 98/03554 et WO 99/36443 et dans le brevet EP 529715 peut s'avérer délicate, en particulier si ces produits possèdent une longue demi-vie. Dans le domaine de la prévention ou du traitement de la thrombose avec les produits ci-dessus, on doit rétablir ou maintenir la fluidité du sang tout en évitant de provoquer une hémorragie.

Il est en effet bien connu que, pour une cause accidentelle quelconque, une hémorragie peut se déclencher chez un patient sous traitement. On peut également avoir besoin d'intervenir chirurgicalement chez un malade sous traitement antithrombotique. De plus, au cours de certains actes chirurgicaux, des anticoagulants peuvent être utilisés à forte dose de façon à empêcher la coagulation du sang, et il est nécessaire de les neutraliser à la fin de l'intervention. Il est donc intéressant d'avoir des agents antithrombotiques neutralisables pour stopper l'activité anticoagulante à tout moment. Or, les oligosaccharides de synthèse connus, décrits précédemment, ne peuvent pas être aisément neutralisés par les antidotes connus de l'héparine ou des HBPM, y compris le sulfate de protamine.

La présente invention concerne de nouveaux hexadécasaccharides biotinylés de synthèse, de structure proche de celle des composés décrits dans la demande de brevet publiée sous le numéro WO 02/24754. Les hexadécasaccharides de l'invention, ainsi que certains composés décrits dans le document WO 02/24754, sont liés de façon covalente avec un dérivé de la biotine (acide hexahydro-2-oxo-*1H*-thiéno[3,4-d]imidazole-4-pentanoïque) via un "spacer" - enchaînements de formule (T₁) ou (T₂) tel que défini dans la présente demande - et possèdent de ce fait l'avantage de pouvoir être rapidement neutralisés par un antidote spécifique, en situation d'urgence. Cet antidote spécifique est l'avidine (« The Merck Index », Twelfth edition, 1996, M.N. 920, pp. 151-152) ou la streptavidine, deux protéines tétramériques de masses respectives égale à environ 66 000 et 60 000 Da, qui possèdent une très forte affinité pour la biotine.

Cependant, et de manière surprenante, il apparaît que la longueur du "spacer" reliant le dérivé de la biotine à la chaîne hexadécasaccharidique, ainsi que la position de la biotine sur l'unité saccharidique sont des facteurs influençant l'efficacité de la neutralisation par un antidote spécifique, et notamment par exemple, par l'avidine. Ainsi, les composés hexadécasaccharidiques selon l'invention présentent une capacité de neutralisation par un antidote spécifique bien supérieure à ceux décrits dans la demande de brevet publiée sous le numéro WO 02/24754, grâce à un "spacer" de taille contrôlée et à la position de la biotine sur l'unité saccharidique.

La présente invention a pour objet des hexadécasaccharides biotinylés de formule générale I: 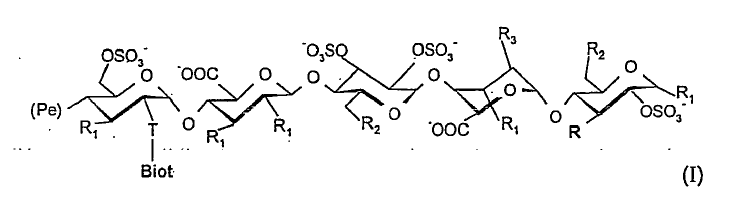 dans laquelle :
- T représente un enchaînement T₁ ou T₂ de formules suivantes : 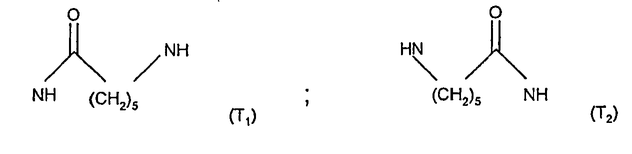
- Biot représente le groupe : 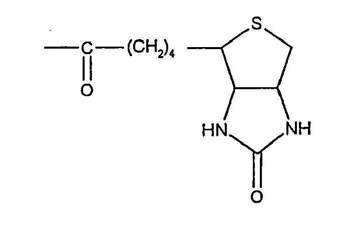
- R représente un radical (C₁-C₆)alcoxy, notamment un radical méthoxy ou un radical -OSO₃⁻,
- R₁ représente un radical (C₁-C₆)alcoxy, notamment un radical méthoxy ou un radical -OSO₃⁻,
- R₂ représente un radical (C₁-C₆)alcoxy ou un radical -OSO₃⁻,
- R₃ représente un radical (C₁-C₆)alcoxy, notamment un radical méthoxy ou un radical -OSO₃⁻, ou bien R₃ constitue un pont -O-CH₂-, le groupe -CH₂- étant lié à l'atome de carbone porteur de la fonction carboxylique sur le même cycle,
- Pe représente un enchaînement saccharidique de formule suivante : 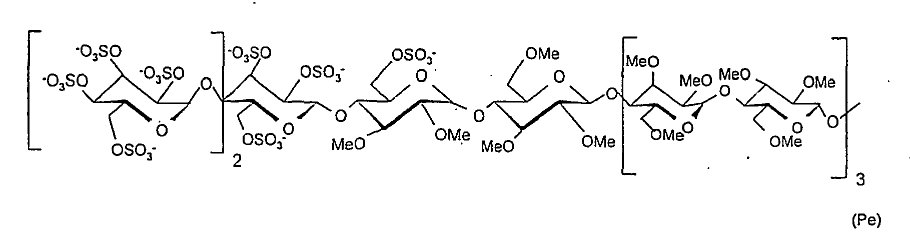
ainsi que leurs sels pharmaceutiquement acceptables.

Les parties polysaccharidiques sont constituées d'unités monosaccharidiques alkylées non chargées et/ou partiellement chargées et/ou totalement chargées. Les unités chargées ou non chargées peuvent être dispersées tout au long de la chaîne ou elles peuvent au contraire être groupées en domaines saccharidiques chargés ou non chargés.

Dans la présente description, il a été choisi de représenter les conformations ¹C₄ pour l'acide-L-iduronique, ⁴C₁ pour l'acide D-glucuronique, mais il est notoire que, d'une façon générale, la conformation en solution des unités monosaccharides est fluctuante.

Ainsi, l'acide L-iduronique peut être de conformation ⁴C₁ ²S₀ ou ⁴C₁.

L'invention englobe les hexadécasaccharides sous leur forme acide ou sous la forme de l'un quelconque de leurs sels pharmaceutiquement acceptables. Dans la forme acide, les fonctions -COO⁻ et -SO₃⁻ sont respectivement sous forme -COOH et -SO₃H.

On entend par sel pharmaceutiquement acceptable des polysaccharides de l'invention, un polysaccharide dans lequel une ou plusieurs des fonctions -COO⁻ ou/et -SO₃⁻ sont liées de façon ionique à un cation pharmaceutiquement acceptable. Les sels préférés selon l'invention sont ceux dont le cation est choisi parmi les cations des métaux alcalins et plus préférablement encore ceux dont le cation est Na⁺ ou K⁺.

Les composés de la formule I ci-dessus comprennent également ceux dans lesquels un ou plusieurs atomes d'hydrogène ou de carbone ont été remplacés par leur isotope radioactif par exemple le tritium ou le carbone C¹⁴. De tels composés marqués sont utiles dans les travaux de recherche, de métabolisme ou de pharmacocinétique, dans les essais biochimiques en tant que ligands.

Dans le cadre de la présente invention, on entend par :
- un radical (C₁-C₆)alcoxy : un radical -O-alkyle, le groupe alkyle étant un radical aliphatique, saturé linéaire ou ramifié, présentant une chaîne de 1 à 6 atomes de carbone: A titre d'exemple de radicaux alkyles, on peut citer les radicaux méthyle, éthyle, propyle, isopropylebutyle, isobutyle, tertbutyle. A titre d'exemple de radicaux (C₁-C₆)alcoxy, on peut citer les radicaux méthoxy, éthoxy,
- un "spacer" T: les enchaînements de formules T₁ ou T₂ suivantes : 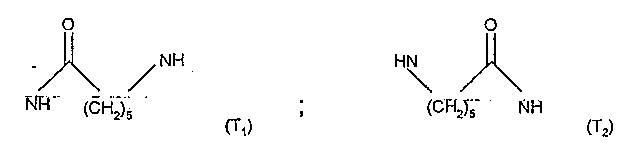
- un dérivé biotinilé :

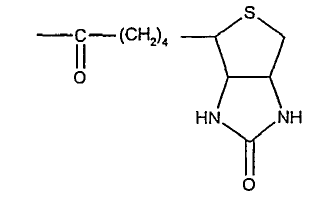

Les dérivés de biotine sont disponibles commercialement (catalogue « Pierce » 1999-2000, pp. 62 à 81).

Selon un de ses aspects préférés, la présente invention concerne les hexadécasaccharidiques biotinylés de formule générale I dans laquelle :
- R représente un radical méthoxy ou un radical - OSO₃⁻,
- R₁ représente un radical méthoxy,
- R₂ représente un radical -OSO₃⁻,
- R₃ représente un radical méthoxy,

Selon un autre de ses aspects particulièrement préférés, l'invention concerne les hexadécasaccharidiques biotinylés suivants :
- Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(→4)-O-(2,3,6-tri-O-méthyl-β-D-glucopyranosyl)-(1→4)]₃-(2-[N-(6-biotinamido hexanoyl)]-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide2,3-di-O-méthyl-α-L-idopyranosyluronique)-(1→4)-3-O-méthyl-2,6-di-O-sulfonato-α-D-glucopyranoside, sel de sodium
- Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-[N-(6-biotinamido hexanoyl)]-2-désoxy-3-O-méthyl-6-O-sulfonato-α-D-glucopyranosyl)-(1→4)-(acide2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

Dans son principe, le procédé de préparation des composés selon l'invention utilise des synthons de base di- ou oligosaccharidiques préparés comme précédemment rapporté dans la littérature. On se référera notamment aux brevets ou demandes de brevet EP 300099, EP 529715, EP 621282 et EP 649854 ainsi qu'aux documents C. van Boeckel, M Petitou, Angew. Chem. Int. Ed. Engl., (1993), 32, pp.1671-1690. Ces synthons sont ensuite couplés les uns aux autres de façon à fournir un équivalent entièrement protégé d'un polysaccharide selon l'invention. Cet équivalent protégé est ensuite transformé en un composé selon l'invention.

L'un des synthons de base évoqués ci-dessus contient une fonction protégée particulière permettant l'introduction ultérieure de la biotine ou d'un dérivé de biotine, par exemple, une fonction amine latente sous forme de groupe azido ou protégée sous forme de N-phtalimido.

Dans les réactions de couplage évoquées ci-dessus, un di- ou oligosaccharide "donneur", activé sur son carbone anomère, réagit avec un di- ou oligosaccharide "accepteur", possédant un hydroxyle libre.

La présente invention concerne un procédé pour la préparation des composés de formule I caractérisé en ce que :
- dans une première étape, on obtient un équivalent complètement protégé de l'hexadécasaccharide de formule I désiré, contenant un précurseur pentasaccharidique protégé, portant notamment une fonction amine convenablement protégée pour l'introduction ultérieure de la biotine ou d'un dérivé de biotine. Ce précurseur pentasaccharidique protégé étant lui-même prolongé par un précurseur protégé du domaine polysaccharidique Pe;
- dans une seconde étape, les groupes chargés négativement sont introduits et/ou démasqués ;
- dans une troisième étape, on déprotège la fonction amine puis on introduit la biotine ou le dérivé de biotine.

La synthèse du pentasaccharide sur lequel sera greffé la biotine ou le dérivé de biotine est réalisée selon les méthodes décrites, en particulier dans les demandes de brevet publiées sous les numéros WO98/03554 et WO99/36443 ainsi que dans la littérature (citée ci-dessus).

La synthèse de la partie polysaccharidique précurseur de Pe est réalisée selon des réactions bien connues de l'homme de l'art, en utilisant les méthodes de la synthèse d'oligosaccharides (G.J. Boons, Tetrahedron, (1996), 52, pp.1095-1121, WO98/03554 et WO99/36443) ou un oligosaccharide lorsqu'un oligosaccharide donneur de liaison glycosidique est couplé avec un oligosaccharide accepteur de liaison glycosidique pour conduire à un autre oligosaccharide dont la taille est égale à la somme des tailles des deux espèces réactives. Cette séquence est répétée jusqu'à l'obtention du composé de formule I. La nature et le profil de la charge du composé final désiré déterminent la nature des entités chimiques utilisées dans les différentes étapes de la synthèse, selon les règles bien connues de l'homme de l'art. On pourra se référer par exemple à C. van Boeckel, M. Petitou, Angew. Chem. Int. Ed. Engl. (1993), 32, pp.1671-1690 ou encore à H. Paulsen, « Advances in selective chemical syntheses of complex oligosaccharides » Angew. Chem. Int. Ed. Engl., (1982), 21, pp.155-173.

Les composés de l'invention sont obtenus à partir de leurs précurseurs polysaccharidiques complètement protégés en utilisant l'enchaînement suivant de réactions :
- les fonctions alcool devant être transformées en un groupe O-sulfo, et les acides carboxyliques sont déprotégés par l'élimination des groupes protecteurs utilisés durant l'élaboration du squelette puis,
- les groupes sulfo sont ensuite introduits,
- la fonction amine permettant d'introduire la biotine ou le dérivé de biotine est déprotégée,
- le dérivé de biotine.est introduite par une réaction classique de couplage amino /acide.

Les composés dé l'invention peuvent naturellement être préparés en utilisant différentes stratégies connues par l'homme de l'art de la synthèse des oligosaccharides.

Le procédé décrit ci-dessus est le procédé préféré de l'invention. Toutefois, les composés de formule I peuvent être préparés par d'autres méthodes bien connues de la chimie des sucres décrites par exemple dans « Monosaccharides, Their chemistry and their roles in natural products », P.M. Collins et R.J. Ferrier, J. Wiley & Sons, (1995) et dans G.J. Boons, Tetrahedron, (1996), 52, p.1095-1121. Les pentasaccharides Pe peuvent donc être obtenus à partir de synthons disaccharidiques de la façon décrite dans la publication de C. van Boeckel, M. Petitou, Angew. Chem. Int. Ed. Engl. (1993), 32, 1671-1690.

Les groupes protecteurs utilisés dans le procédé de préparation des composés de formule I sont ceux couramment utilisés dans la chimie des sucres, par exemple dans « Protective Groups in Organic Synthesis », (1981), TW Greene, John Wiley & sons, New-York.

Les groupes protecteurs sont avantageusement choisis, par exemple parmi les groupes acétyles, halogénométhyles, benzoyles, lévulinyles, benzyles, benzyles substitués, trityles éventuellement substitués, tétrahydropyranyles, allyles, pentenyles, tert-butyldiméthylsilyles (tBDMS) ou triméthylsilyléthyles.

Les groupes activateurs sont ceux classiquement utilisés en chimie des sucres selon par exemple G.J. Boons, Tetrahedron, (1996), 52, pp.1095-1121. Ces groupes activateurs sont choisis par exemple parmi les imidates, les thioglycosides, les penténylglycosides, les xanthates, les phosphites ou les halogénures.

En ce qui concerne et la façon dont le dérivé de biotine est liée à l'oligosaccharide ainsi que la nature du dérivé de biotine, la littérature chimique offre d'autres possibilités qu'il est possible de mettre en oeuvre par des jeux de groupes protecteurs bien connus de l'homme de l'art. On utilisera de préférence une fonction amine, ou encore une fonction thiol, ou encore une fonction acide carboxylique ou encore une fonction aldéhyde que l'on fera réagir avec un dérivé de biotine comportant un groupe réactif du type ester activé, maléimide, iodoacétyl ou amine primaire, la réaction se faisant selon les conditions décrites dans la littérature (Savage et al., « Avidin-Biotin Chemistry: A Handbook »; (1992), Pierce Chemical Company).

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir le sel souhaité. Pour la préparation des sels des composés de formule I, on peut utiliser toute base minérale ou organique, donnant avec les composés de formule I, des sels pharmaceutiquement acceptables. On utilise de manière préférentielle comme base l'hydroxyde de sodium, de potassium, de calcium ou de magnésium. Les sels de sodium et de calcium des composes de formule I, sont les sels préférés.

Les composés selon l'invention ont fait l'objet d'études biochimiques et pharmacologiques.

### 1. Mesure de l'activité anti-facteur IIa et de l'activité anti-facteur Xa

L'activité circulante des composés selon l'invention peut être mesurée à l'aide de leur activité anti-facteur IIa et l'activité anti-facteur Xa comme décrite par Herbert et al., Thromb Haemost., (2001), 85(5), pp. 852-60. Les composées selon l'invention sont administrés par voie intraveineuse (IV) ou sous-cutanée (SC) chez le rat.

Une injection d'avidine IV entraîne une diminution importante de la concentration circulante de composé selon l'invention (supérieur à 70%).

Par exemple, la concentration circulante du composé selon l'exemple 1, après injection IV de 100 nmol/kg est diminuée de 88 % (diminution mesurée via l'activité anti-facteur Xa) et 91 % (diminution mesurée via l'activité anti-facteur IIa), 2 minutes après l'administration IV d'avidine (10mg/kg/625 nmol/kg).

La concentration circulante du composé selon l'exemple 2, après injection IV de 100 nmol/kg est diminuée de 76 % (diminution mesurée via l'activité anti-facteur Xa) et 89 % (diminution mesurée via l'activité anti-facteur IIa), 5 minutes après l'administration IV d'avidine (10mg/kg / 625 nmol/kg).

Des inhibitions équivalentes pour les deux activités sont observés quand les composés selon les exemples 1 et 2 sont administrés SC ;

### 2. Mesure de l'activité antithrombotique globale et neutralisation par l'avidine

L'activité antithrombotique globale des composés selon l'invention et leur neutralisation a été étudiée dans un modèle de thrombose veineuse constitué d'une injection de facteur tissulaire suivie d'une stase de la veine cave de rats, tels que décrits par Herbert et al., Blood, (1998), 91, pp. 4197-4205.

### a) Mesure de l'activité antithrombotique globale

Les composés de la présente invention sont des puissants inhibiteurs de la, thrombose (valeurs d'IC₅₀ inférieure à 50 nM).

Par exemple les exemples 1 et 2 montrent des IC₅₀ dans ce modèle de 3 et 9.9 nM respectivement après leur administration IV.

Par exemple, l'inhibition du poids du thrombus due au composé selon l'exemple 1 (à une concentration de 30 nmol/kg), est ramenée au niveau du contrôle par une injection IV de 3 mg/kg/208 nmol/kg d'avidine.

### b) Neutralisation par l'avidine : test de saignement chez le rat

L'effet de composés selon l'invention a été évalué dans le test de saignement chez le rat (Herbert et al., Blood, (1998), 91, pp. 4197-4205). Ces composés présentent une forte activité anticoagulante et augmentent donc le temps de saignement dans les modèles animaux. L'avidine neutralise l'effet des composés selon l'invention sur le saignement.

Par exemple, le temps de saignement induit chez le rat par 30 nmol/kg du composé selon l'exemple 1, est ramené au niveau du contrôle par une administration IV d'avidine (3 mg/kg ; 208 nmol/kg).

Par exemple, le temps de saignement induit chez le rat par 100 nmol/kg du composé selon l'exemple 2, est ramené au niveau du contrôle par une administration IV d'avidine (10 mg/kg ; 625 nmol/kg).

Ainsi, la présente invention a également pour objet un procédé mettant en oeuvre l'avidine ou la streptavidine caractérisé en ce qu'il permet de neutraliser les polysaccharides selon l'invention. L'avidine ou la streptavidine peuvent être utilisées pour la préparation de médicaments destinés à neutraliser les polysaccharides selon la présente invention.

Grâce à leur activité biochimique et pharmaceutique, les oligosaccharides de la présente invention constituent des médicaments très intéressants. Leur toxicité est parfaitement compatible avec cette utilisation. Ils sont également très stables et sont donc particulièrement appropriés pour constituer le principe actif de spécialités pharmaceutiques.

Ils peuvent être utilisés dans diverses pathologies consécutives à une modification de l'homéostasie du système de la coagulation apparaissant en particulier lors des troubles du système cardio-vasculaire et cérébro-vasculaire comme les troubles thromboemboliques associés à l'arthérosclérose et au diabète tels que l'angine instable, l'attaque cérébrale, la resténose après angioplastie, l'endartérectomie, la pose de prothèses endovasculaires ; ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens. Ces produits peuvent par ailleurs être utilisés pour le traitement ou la prévention de pathologies thromboemboliques d'origine veineuse telles les embolies pulmonaires. Ils peuvent être utilisés ou pour prévenir ou pour traiter les complications thrombotiques observés par exemple à la suite d'opérations chirurgicales, de développements tumoraux ou de dérèglements de la coagulation, induits par des activateurs bactériens, viraux, ou enzymatiques: Dans le cas de leur utilisation lors de la pose de prothèses, les composés de la présente invention peuvent recouvrir des prothèses et les rendre ainsi hémocompatibles. En particulier, ils peuvent être fixés à des prothèses intravasculaires (stents). Dans ce cas, ils peuvent éventuellement être modifiés chimiquement par introduction à l'extrémité non réductrice ou réductrice d'un bras approprié, comme décrit selon EP 649854.

Les composés de la présente invention peuvent également être utilisés comme adjuvants lors d'endartérectomie réalisée avec des ballonnets poreux.

Les composés selon l'invention peuvent être utilisés pour la préparation de médicaments destinés à traiter les maladies ci-dessus.

Selon un autre de ses aspects, la présente invention a donc pour objet une composition pharmaceutique contenant, en tant que principe actif, un polysaccharide de synthèse selon l'invention ou un de ses sels pharmaceutiquement acceptables, éventuellement en association avec un ou plusieurs excipients inertes et appropriés.

Lesdits excipients sont choisis selon la forme pharmaceutique et le mode d'administration souhaités : orale, sublinguale, sous-cutanée, intramusculaire, intraveineuse, transdermique, transmuqueux, locale ou rectale.

Le principe actif peut être également présenté sous forme de complexe avec une cyclodextrine, par exemple α, β ou y cyclodextrine, 2-hydroxypropyl-β-cyclodextrine ou méthyl-β-cyclodextrine.

Le principe actif peut également être libéré par un ballonnet le contenant ou par un extenseur endovasculaire introduit dans les vaisseaux sanguins. L'efficacité pharmacologique du principe actif n'est ainsi pas affectée.

Dans chaque unité de dosage, le principe actif est présent dans les quantités adaptées aux doses journalières envisagées afin d'obtenir l'effet prophylactique ou thérapeutique désiré. Chaque unité de dosage peut contenir de 0,1 à 100 mg de principe actif, de préférence 0,5 à 50 mg. Ces doses de composés anticoagulants pourraient être neutralisées par des doses d'avidine ou de streptavidine allant de 1 à 1000 mg en injection intraveineuse bolus ou perfusion.

Les composés selon l'invention peuvent également être utilisés en association avec un ou plusieurs autres principes actifs utiles pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoprotéine IIb/IIIa.

Les méthodes, les préparations et les schémas suivants illustrent la synthèse des différents intermédiaires utiles à l'obtention des polysaccharides selon l'invention. Les exemples de synthèse d'hexadécasaccharides qui suivent illustrent l'invention sans la limiter. Les abréviations suivantes sont utilisées :
Bn : benzyle ;
Bz : benzoyle ;
Lev : lévulinyl ;
Et : éthyle ;
Ph : phényle ;
Me : méthyle ;
Ac : acétyle ;
SEt : thioéthyle;
MP : p-méthoxyphényle ;
Biotine : Acide hexahydro-2oxo-1H-thiéno[3,4-d]imidazole-4-pentanoïque;
ESI : est le sigle anglais Electron Spray Ionisation ;
CCM : chromatographie sur couche mince ;
PF : pont de fusion ;
[α_{D]} = pouvoir rotatoire ;
C = concentration ;
Rf = temps de rétention mesuré sur la CCM par rapport au front du solvant de migration.

Dans la suite, les préparations et des exemples de synthèse des composés de l'invention sont détaillés à titre d'illustration.

### PREPARATIONS

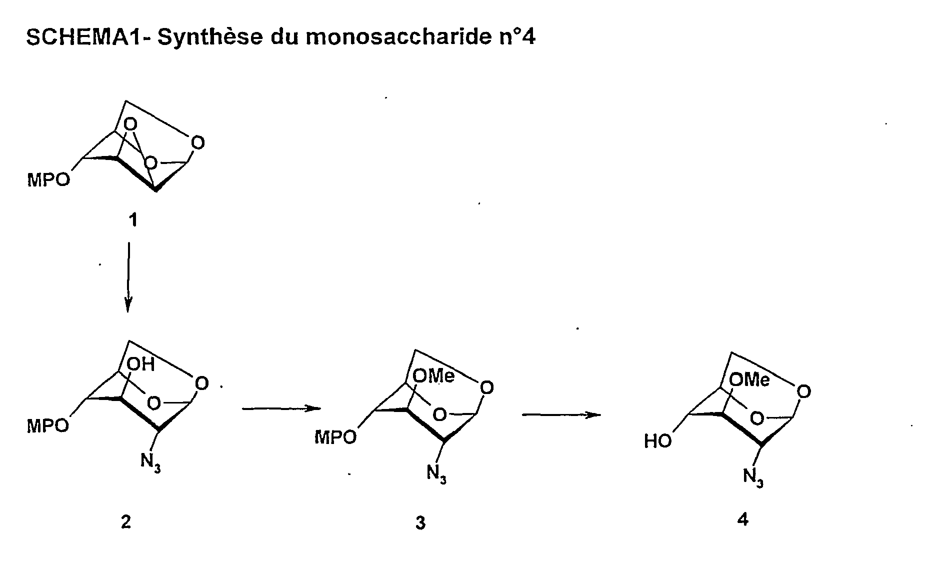

### PREPARATION 1

### Préparation du 1,6-Anhydro-2-azido-2-désoxy-4-O-p-méthoxyphényl-β-D-glucopyranose (n° 2)

Le composé 1,6:2,3-dianhydro-4-O-p-méthoxyphényl-β-D-mannopyranose, n° 1, (4.39 g, 17,5 mmol) est synthétisé par analogie avec la méthode décrite dans Brill and Tirefort, Tetrahedron Lett. (1998), 39, pp: 787-790. Le composé n° 1 est dissous dans 130 mL d'un mélange N,N-diméthylformanide/eau [4/1 (v/v)] puis de l'azoture de sodium (22,8 g, 350 mmol) est ajouté. Le milieu réactionnel est chauffé à 120 °C pendant 6 heures. Après filtration sur Célite, le filtrat est dilué avec de l'acétate d'éthyle puis lavé avec de l'eau. La phase organique est séchée sur sulfate de sodium, filtrée puis concentrée sous vide. Le résidu est recristallisé dans un mélange acétate d'éthyle/cyclohexane (20mL/7 mL) pour donner 4,46 g du composé n° 2 sous forme de cristaux.
PF : 144 °C

### PREPARATION 2

### Préparation du 1,6-Anhydro-2-azido-2-désoxy-4-O-p-méthoxyphényl-3-O-méthyl-β-D-glucopyranose (n° 3)

A un mélange refroidi (0°C) du composé n° 2 (4,08 g, 13,9 mmol), d'iodure de méthyle (1,1 mL, 15,3 mmol) dans du N,N-diméthylformamide anhydre (40 mL), on ajoute, par petites quantités, de l'hydrure de sodium (1,04 g) sous atmosphère d'argon. Le mélange est agité pendant 20 heures à température ambiante. L'excès d'hydrure de sodium est détruit avec du méthanol. Après évaporation du N,N-diméthylformamide, le résidu est repris avec du dichlorométhane. La phase organique est lavée avec de l'eau, séchée sur sulfate de sodium, filtrée puis concentrée sous vide. Le résidu est purifié par chromatographie sur colonne de gel de silice [toluène/acétate d'éthyle 12/1 (v/v)] pour donner 3,57 g du composé n° 3 sous forme d'un solide blanc.
PF : 68 °C

### PREPARATION 3

### Préparation du 1,6-Anhydro-2-azido-2-désoxy-3-O-méthyl-β-D-glucopyranose (n° 4)

Une solution du composé n° 3 (8,0 g, 26,03 mmol) dans 130 mL d'un mélange acétonitrile/eau [9/1 (v/v)] est ajoutée goutte à goutte à une solution de cérium ammonium nitrate (86 g, 156,2 mmol) dans 390 mL d'un mélange acétonitrile/eau [9/1 (v/v)]. Le mélange est agité à température ambiante pendant 40 minutes puis dilué avec de l'acétate d'éthyle. Le mélange réactionnel est séché sur sulfate de sodium anhydre, filtré et concentré. Une filtration sur gel de silice permet en partie d'éliminer les résidus de cérium ammonium nitrate. Pour purifier le composé, une acétylation suivie d'une désacétylation seront réalisées. Le résidu est repris dans du dichlorométhane (90 mL) puis successivement sont ajoutés de la triéthylamine (5,3 mL), de la diméthylaminopyridine (280 mg) et enfin de l'anhydride acétique (3,2 mL). Après 16 heures, le milieu réactionnel est dilué avec du dichlorométhane (250 mL). La phase organique est lavée avec une solution d'hydrogénosulfate de potassium à 10%, de l'eau, une solution d'hydrogénocarbonate de sodium à 10 % puis de l'eau. La phase organique est ensuite séchée sur sulfate de sodium anhydre, filtrée puis concentrée sous vide. Le résidu est purifié par chromatographie sur colonne de gel de silice [toluène/acétate d'éthyle 10/1 (v/v)] pour donner 4,9 g du produit sous forme de solide. Ce solide est dissous dans 80 mL d'un mélange dichlorométhane/méthanol [1/1 (v/v)] puis est ajouté du méthylate de sodium (544 mg). Le mélange est agité pendant 35 minutes puis neutralisé avec une résine Dowex^{®} 50WX4 H⁺, filtré et concentré sous vide. Le résidu est purifié par chromatographie sur colonne de gel de silice [toluène/acétate d'éthyle 3/2 (v/v)] pour donner 3,9 g du composé n°4.
[α]_{D} = -27°(C = 1,06, dans le dichlorométhane).

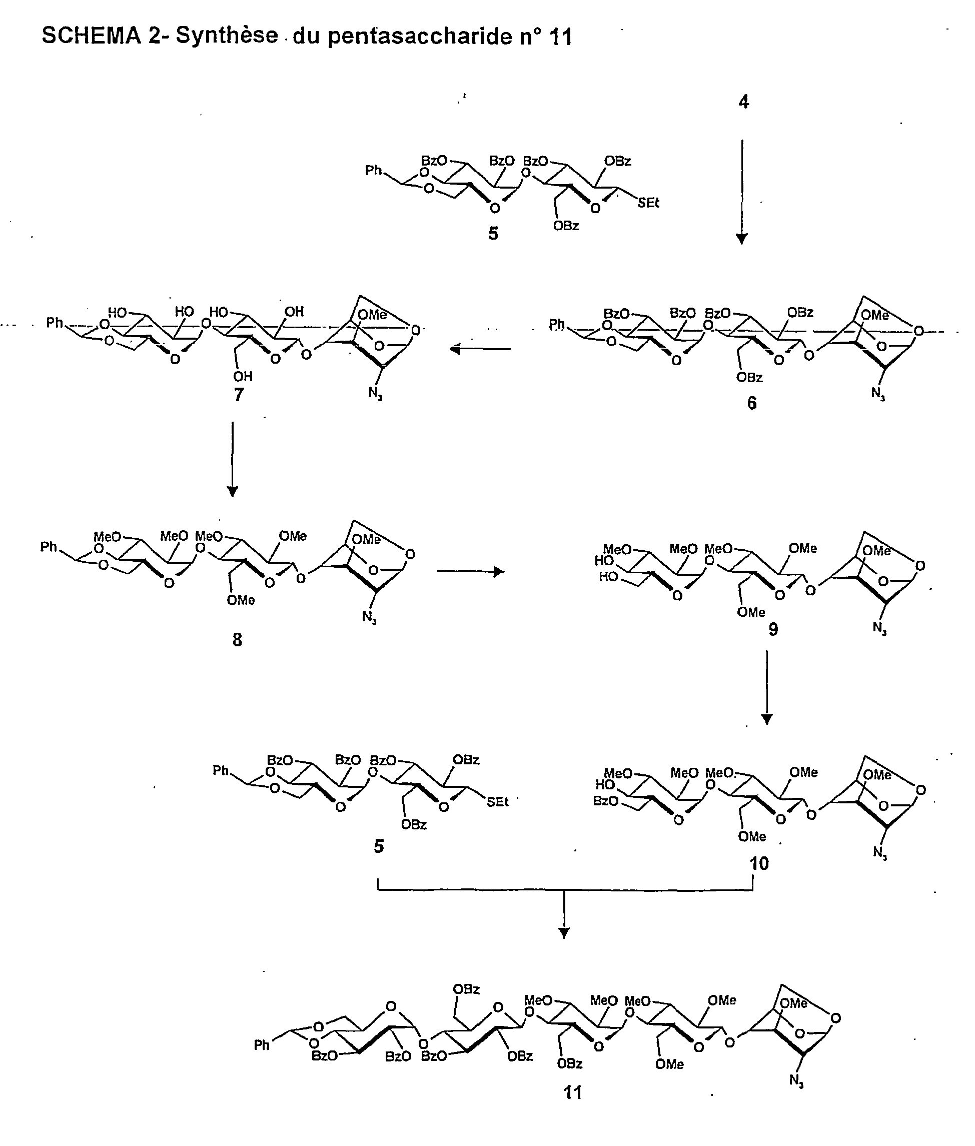

### PREPARATION 4

### Préparation du (2,3-Di-O-benzoyl-4,6-O-benzylidène-α-D-glucopyranosyl)-(1→4)-(2,3,6-tri-O-benzoyl-β-D-glucopyranosyl)-(1→4)-1,6-anhydro-2-azido-2-désoxy-3-O-Méthyl-β-D-glucopyranose (n° 6)

Un mélange du composé thioglycoside n° 5 (9,00 g, 9,04 mmol), obtenu par analogie avec la préparation 1 décrite dans de la demande de brevet publiée sous le numéro WO 99/36443, du composé n° 4 obtenu à la PREPARATION 3 (1,65 g, 8,22 mmol) et de tamis moléculaire 4Å en poudre (9,05 g) dans le toluène (180 mL) est agité sous atmosphère d'argon pendant 1 heure. Puis le mélange est refroidi à - 20 °C. Une solution de N-iodosuccinimide (2,14 g, 9,5 mmol) et d'acide trifluorométhane sulfonique (96 µL, 1,09 mmol) dans 47 mL d'un mélange dichlorométhane/dioxane [1/1 (v/v)], est ajoutée goutte à goutte au mélange réactionnel. Après 10 minutes, le mélange réactionnel est filtré sur Célite, dilué avec du dichlorométhane (1000 mL), successivement lavé avec une solution de thiosulfate de sodium 1 M, une solution d'hydrogénocarbonate de sodium à 10% et de l'eau. Le mélange réactionnel est ensuite séché sur sulfate de sodium anhydre puis concentré sous vide. La purification du résidu est effectuée par chromatographie sur colonne de gel de silice [toluène/acétate d'éthyle 5/1 (v/v)] pour donner 9,20 g du trisaccharide n° 6.
[α]_{D} = +44° (C = 1,30, dans le dichlorométhane).

### PREPARATION 5

### Préparation du (4,6-O-Benzylidène-α-D-glucopyranosyl)-(1→4)-(β-D-glucopyranosyl)-(1→4)-1,6-anhydro-2-azido-2-désoxy-3-O-méthyl-β-D-glucopyranose (n° 7)

À une solution du composé n° 6 (9,2 g, 8,11 mmol) dans du dioxane (81 ml), on ajoute du tert-butylate de potassium (1,82 g, 16,2 mmol). Le mélange est agité pendant 3 heures puis neutralisé avec une résine Dowex^{®} 50WX4 H⁺, filtré et concentré sous vide. Après une chromatographie sur colonne de gel de silice [dichlorométhane/méthanol 8/1 (v/v)], 5,46 g du composé n° 7 sont isolés sous forme de mousse.
CCM sur gel de silice, dichlorométhane/méthanol [9/1 (v/v)] : Rf = 0,35
[α]_{D} = +38° (C = 0,84 dans le dichlorométhane).

### PREPARATION 6

### Préparation du (4,6-O-Benzylidène-2,3-di-O-méthyl-α-D-glucopyranosyl)-(1→4)-(2,3,6-tri-O-méthyl-β-D-glucopyranosyl)-(1→4)-1,6-anhydro-2-azido-2-désoxy-3-O-méthyl-β-D-glucopyranose (n° 8)

A un mélange refroidi (0°C) du composé n° 7 (5.05 g, 8,23mmol), d'iodure de méthyle (3;8 mL, 61,7 mmol) dans du N,N-diméthylformamide anhydre (150 mL), on ajoute, par petites quantités, de l'hydrure de sodium (1,73 g, 72,0 mmol) sous atmosphère d'argon. Le mélange est agité pendant 20 heures à température ambiante. L'excès d'hydrure de sodium est détruit avec du méthanol (8 mL) et le mélange réactionnel est versé dans l'eau glacée (400 mL). Après extraction avec de l'acétate d'éthyle, la phase organique est lavée avec une solution saturée de chlorure de sodium, séchée sur sulfate de sodium anhydre puis concentrée sous vide. La purification du résidu est effectuée par chromatographie sur colonne de gel de silice [dichlorométhane/acétone 7/1 puis 5/1 (v/v)] pour donner 4,8 g du composé n° 8.
[α]_{D} = +49° (C = 1,02, dans le dichlorométhane).
CCM sur gel de silice, dichlorométhane/acétone [5/1 (v/v)] : Rf = 0,45

### PREPARATION 7

### Préparation du (2,3-Di-O-méthyl-α-D-glucopyranosyl)-(1→4)-(2,3,6-tri-O-méthyl-β-D-glucopyranosyl)-(1→4)-1,6-anhydro-2-azido-2-désoxy-3-O-méthyl-β-D-glucopyranose (n° 9)

Le composé n° 8 (5,3 g, 7,75 mmol) est dissous dans de l'acide acétique à 60% (233 mL) et agité pendant 1 heure 30 minutes à 80 °C. Le mélange est concentré et co-évaporé avec du toluène. Le résidu est purifié par chromatographie sur colonne de gel de silice [toluène/éthanol 4/1 (v/v)] pour donner 5,09 g du composé n°9.
[α]_{D} = +57° (C = 1,06, dans le dichlorométhane).
CCM sur gel de silice, toluène/éthanol [4/1 (v/v)] : Rf = 0,36

### PREPARATION 8

### Préparation du (6-O-Benzoyl-2,3-di-O-méthyl-α-D-glucopyranosyl)-(1→4)-(2,3,6-tri-O-méthyl-β-D-glucopyranosyl)-(1→4)-1,6-anhydro-2-azido-2-désoxy-3-O-méthyl-β-D-glucopyranose (n° 10)

A une solution du composé n° 9 (5,09 g, 8,55 mmol) dans du dichlorométhane (85 mL), on ajoute du 1-benzoyloxy-1*H*-benzotriazole (2,86 g, 11,97 mmol) et de la triéthylamine (1,80 mL). Le mélange est agité pendant 20 heures à température ambiante, puis dilué avec du dichlorométhane. La phase organique est lavée avec une solution saturée d'hydrogénocarbonate de sodium puis de l'eau. La phase organique est ensuite séchée sur sulfate de sodium anhydre, filtrée puis concentrée sous vide. Le résidu est purifié par chromatographie sur colonne de gel de silice [toluène/éthanol 15/2 (v/v)] pour donner 4,85 g du composé n° 10.
[α]_{D} = +43° (C = 1,06, dans le dichlorométhane).
CCM sur gel de silice, toluène/éthanol [15/2 (v/v)] : Rf = 0,31

### PREPARATION 9

### Préparation du (2,3-Di-O-benzoyl-4,6-O-benzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°11)

La réaction de couplage du composé n° 10 (4,38 g, 6,26 mmol) avec le composé n° 5 (« donneur de glycosyle supprimé »), obtenu par analogie avec la préparation 1 décrite dans la demande de brevet publiée sous le numéro WO 99/36443 (11,85 g, 11,9 mmol) est réalisée selon le mode opératoire décrit à la PREPARATION 4, pour donner 8,39 g du composé n° 11.
[α]_{D} = +61° (C= 1,06, dans le dichlorométhane).

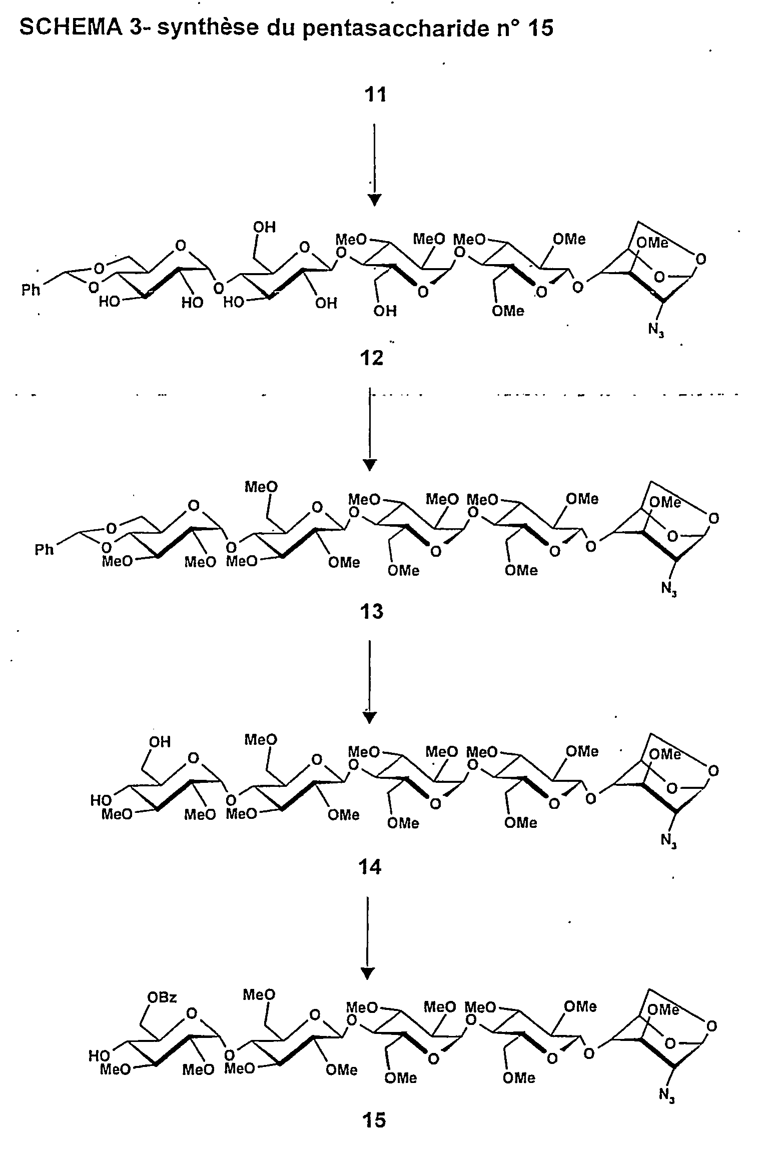

### PREPARATION 10

### Préparation du (4,6-O-Benzylidène-α-D-glucopyranosyl)-(1→4)-(β-D-glucopyranosyl)-(1→4)-(2,3-di-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n° 12)

Le composé n° 11 (8,36 g, 5,12 mmol) obtenu à la préparation 9, est transformé en composé 12. selon le même mode opératoire que celui décrit pour la PREPARATION 5. Après chromatographie sur colonne de gel de silice, le composé n° 12 (4,92 g) est obtenu sous forme de verre.
CCM sur gel de silice, dichlorométhane/méthanol [10/1 (v/v)]: Rf = 0,41

### PREPARATION 11

### Préparation du (4,6-O-Benzylidène-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°13)

Le composé n° 12 (4,57 g, 4,54 mmol) est transformé en composé n° 13 selon le même mode opératoire que celui décrit pour la PREPARATION 6. Le brut est purifié par chromatographie sur colonne de gel de silice afin d'obtenir 4,94 g du composé n° 13.
[α]_{D} = +68 °(C = 0,93, dans le dichlorométhane)
CCM sur gel de silice, toluène/éthanol [8/1 (v/v)] : Rf = 0,39

### PREPARATION 12

### Préparation du (2,3-Di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°14)

Le composé n° 13 (4,89 g, 4,48 mmol) est transformé en composé n° 14 selon le même mode opératoire que celui décrit pour la PREPARATION 7. Le brut est purifié par chromatographie sur colonne de gel de silice afin d'obtenir 4,30 g du composé n° 14.
[α]_{D} = + 80 °(C = 1,05, dans le dichlorométhane)
CCM sur gel de silice, toluène/éthanol [4/1 (v/v)] : Rf = 0,31

### PREPARATION 13

### Préparation du (6-O-Benzoyl-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°15)

Le composé n° 14 (4,26 g, 4,25 mmol) est transformé en composé n° 15 selon le même mode opératoire que celui décrit pour la PREPARATION 8. Le brut est purifié par chromatographie sur colonne de gel de silice afin d'obtenir 4,33 g du composé n° 15.
[α]_{D} = +59°(C = 1,0, dans le dichlorométane)
CCM sur gel de silice, toluène/acétone [4/3 (v/v)] Rf = 0,38

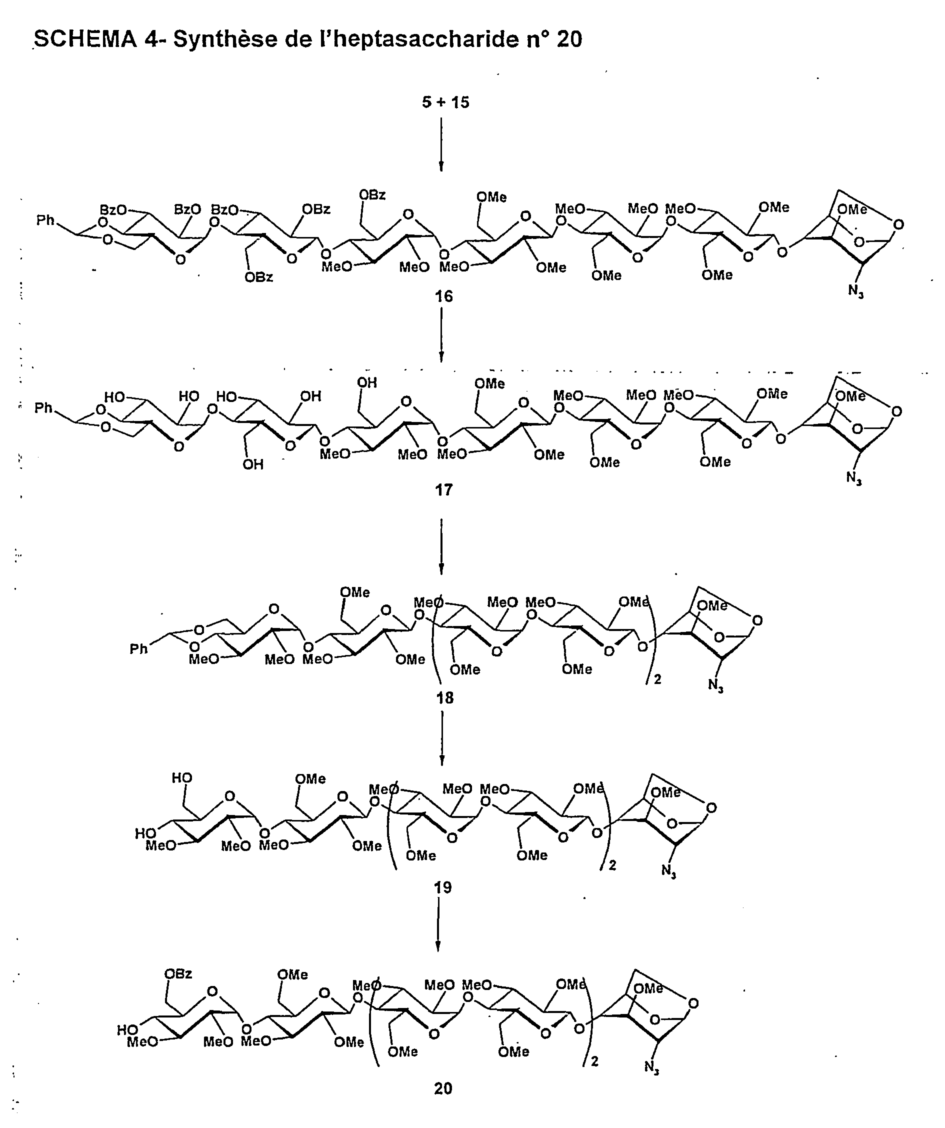

### PREPARATION 14

### Préparation du (2,3-Di-O-benzoyl-4,6-O-benzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°16)

La réaction de couplage du thioglycoside n° 5 (4,90 g, 4,93 mmol), obtenu par analogie avec la préparation décrite dans la demande de brevet publiée sous le numéro WO 99/36443, et du composé n° 15 (4,55 g, 4,11 mmol), décrit à la PREPARATION 13, est réalisée selon le mode opératoire décrit à la PREPARATION 4. Le résidu obtenu après extraction est purifié par : chromatographie sur colonne de gel de silice pour donner 8,07 g du composé n°16.
[α]_{D} = +71° (C = 0,99, dans le dichlorométhane).

### PREPARATION 15

### Préparafion du (4,6-O-Benzylidène-α-D-glucopyranosyl)-(1→4)-(β-D-glucopyranosyl)-(1→4)-(2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-1,6-anhydro-2-azido-2-désoxy-3-O-méthyl-β-D-glucopyranose (n°17)

Le composé n° 16 est transformé en composé n° 17 selon le mode opératoire décrit à la PREPARATION 5.
CCM sur gel de silice, dichlorométhane/méthanol [8/1 (v/v)] : Rf = 0,45

### PREPARATION 16

### Préparation du (4,6-O-Benzylidène-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₂-1,6-anhydro-2-azido-2-désoxy 3-O-méthyl-β-D-glucopyranose (n° 18)

Le composé n° 17 est transformé en composé n° 18 selon le mode opératoire décrit à la PREPARATION 6.
CCM sur gel de silice, toluène/acétone [5/4 (v/v)] : Rf = 0,40

### PREPARATION 17

### Préparation du (2,3-Di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₂-1,6-anhydro-2-azido-2-désoxy-3-O-méthyl-β-D-glucopyranose (n° 19)

Le composé n° 18 est transformé en composé n° 19 selon le mode opératoire décrit à la PREPARATION 7.
CCM sur gel de silice, dichlorométhane/méthanol [10/1 (v/v)] : Rf = 0,41

### PREPARATION 18

### Préparation du (6-O-Benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₂-1,6-anhydro-2-azido-2-désoxy-3-O-méthyl-β-D-glucopyranose (n° 20)

Le composé n° 19 est transformé en composé n° 20 selon le mode opératoire décrit à la PREPARATION 8.
CCM sur gel de silice, cyclohexane/acétone [1/1 (v/v)] : Rf = 0,36

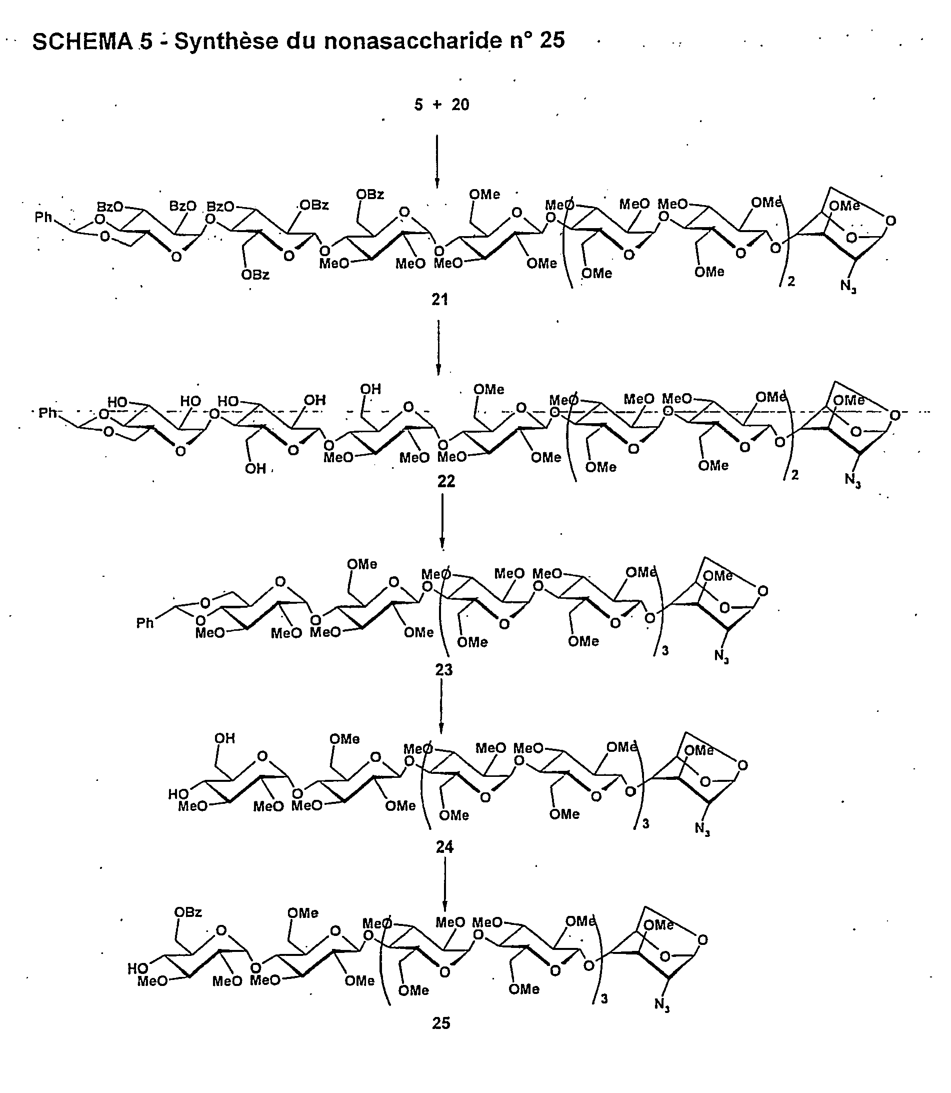

### PREPARATION 19

### Préparation du (2,3-Di-O-benzoyl-4,6-O-benzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)]₂-(1→4)-1,6-anhydro-2-azido-2-désoxy-3-O-méthyl-β-D-glucopyranose (n° 21)

La réaction de couplage du thioglycoside n°5 (3,91 g, 3,93 mmol), obtenu par analogie avec la préparation 1 décrite dans la demande de brevet publiée sous le numéro WO 99/36443, et du composé n° 20 (4,97 g, 3,27 mmol), obtenu à la préparation 18, est réalisée selon le mode opératoire décrit à la PREPARATION 4. Le brut est purifié par chromatographie sur colonne de gel de silice pour donner 8,06 g du composé n° 21.
[α]_{D} = +77 °(C = 0,92, dans le dichlorométhane)

### PREPARATION 20

### Préparation du (4,6-O-Benzylidène-α-D-glucopyranosyl)-(1→4)-(β-D-glucopyranosyl)-(1→4)-(2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)]₂-(1→4)-1,6-anhydro-2-azido-2-désoxy-3-O-méthyl-β-D-glucopyranose (n° 22)

Le composé n° 21 est transformé en composé n° 22 selon le mode opératoire décrit à la PREPARATION 5.
CCM sur gel de silice, dichlorométhane/méthanol [9/1(v/v)] : Rf = 0,34

### PREPARATION 21

### Préparation du (4,6-O-Benzylidène-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)]₃-(1→4)-1,6-anhydro-2-azido-2-désoxy-3-O-méthyl-β-D-glucopyranose (n° 23)

Le composé n° 22 est transformé en composé n° 23 selon le mode opératoire décrit à la PREPARATION 6.
CCM sur gel de silice, cyclohexane/acétone [1/1 (v/v)] : Rf = 0,44

### PREPARATION 22

### Préparation du (2,3-Di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)]₃-(1→4)-1,6-anhydro-2-azido-2-désoxy-3-O-méthyl-β-D-glucopyranose (n° 24)

Le composé n° 23 est transformé en composé n° .24 selon le mode opératoire décrit à la PREPARATION 7.
CCM sur gel de silice; dichlorométane/méthanol [10/1 (v/v)] : Rf = 0,43

### PREPARATION 23

### Préparation du (6-O-Benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)]₃-(1→4)-1,6-anhydro-2-azido-2-désoxy-3-O-méthyl-β-D-glucopyranose (n° 25)

Le composé n° 24 est transformé en composé n° 25 selon le mode opératoire décrit à la PREPARATION 8.
CCM sur gel de silice, dichlorométane/méthanol [10/1 (v/v)] : Rf = 0,64

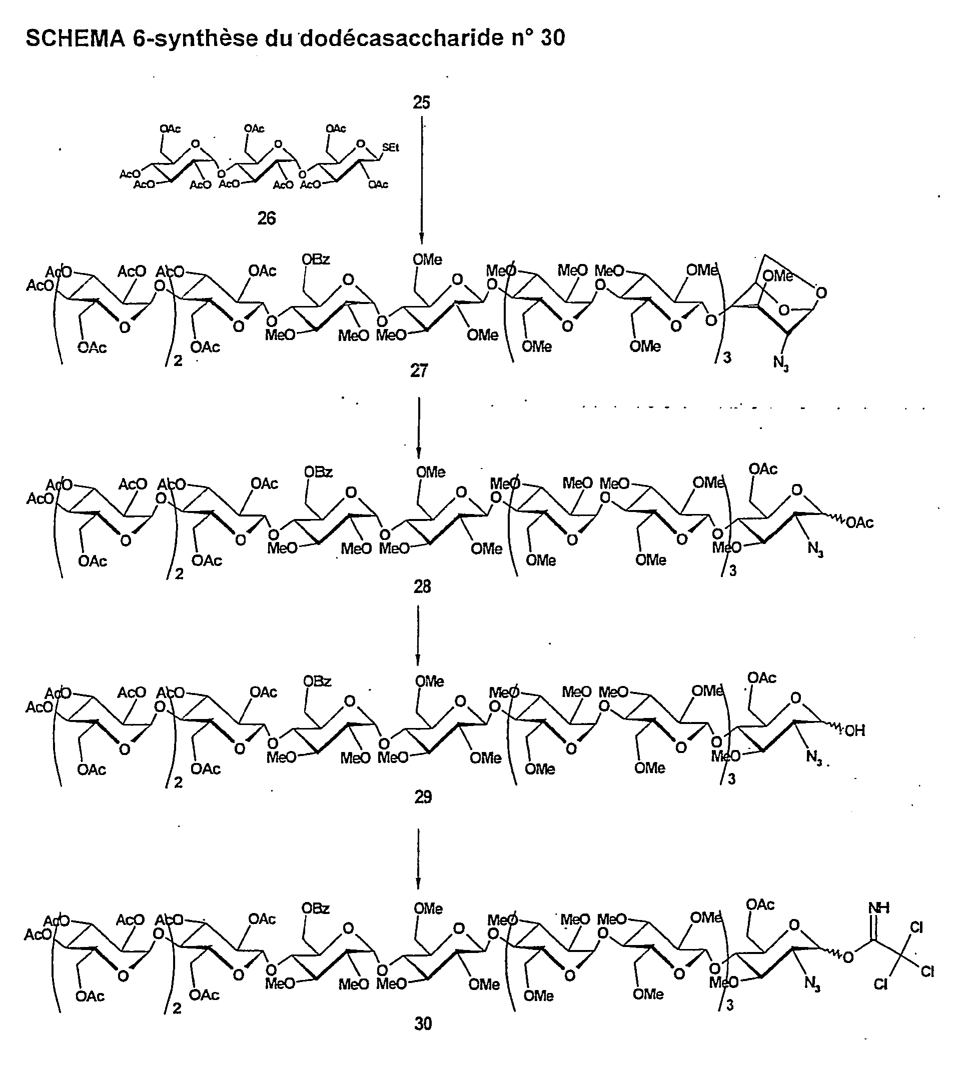

### PREPARATION 24

### Préparation du (2,3,4,6-Tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-1,6-anhydro-2-azido-2-désoxy-3-O-méthyl-β-D-glucopyranose (n° 27)

Un mélange d'un composé thioglycoside n° 26 (10,1 g, 10,4 mmol), préparé par analogie avec la méthode décrite dans la préparation 36 de la demande de brevet publiée sous le n° WO 99/36443, du composé accepteur n° 25 (5,02 g, 2,61 mmol) obtenu à la PREPARATION 23 et de tamis moléculaire 4Å en poudre (14,5 g) dans le toluène (220 mL) est agité sous atmosphère d'argon pendant 1 heure. Le mélange réactionnel est refroidi à 0°C et une solution de N-iodosuccinimide (2,58 g) et d'acide trifluorométhanesulfonique (366 µL) dans 57 mL d'un mélange dichlorométhane/dioxane [1/1 (v/v)] y est introduite. Après 40 minutes, le mélange est filtré sur Célite, dilué avec du toluène, lavé successivement avec une solution de thiosulfate de sodium 1 M, une solution d'hydrogénocarbonate de sodium à 10% et de l'eau. Le mélange réactionnel est ensuite séché sur sulfate de sodium anhydre, filtré puis concentré sous vide. Le résidu est purifié par chromatographie sur colonne de gel de silice [dichlorométhane/acétate d'éthyle/éthanol 17/1/1 puis 14/1/1 (v/v/v)] et donne 5,87 g du composé n° 27.
CCM sur gel de silice, dichlorométhane/acétate d'éthyle/éthanol [8/0,5/0,5 (v/v/v)] ; [toluène/acétone (1/1 (v/v)] : Rf = 0,41

### PREPARATION 25

### Préparation du (2,3,4,6-Tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-1,6-di-O-acétyl-2-azido-2-désoxy-3-O-méthyl-D-glucopyranose (n° 28)

Une solution du composé n° 27 (4,06 g, 1,44 mmol) dans un mélange d'anhydride acétique (13,6 mL) et d'acide trifluoroacétique (1,2 mL) est agitée pendant 6 heures. Après concentration, le mélange est co-évaporé avec du toluène (5 x 25 mL). La purification de résidu par chromatographie sur colonne de gel de silice [cyclohexane/acétate d'éthyle/éthanol 3/1/1 (v/v/v)] donne 2,930 g du composé n° 28.
CCM sur gel de silice, toluène/acétone [1/1 (v/v)] : Rf = 0,44

### PREPARATION 26

### Préparation du (2,3,4,6-Tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-methyl-β-D-glucopyranosyl)-(1→4)]₃-6-O-acétyl-2-azido-2-désoxy-3-O-méthyl-D-glucopyranose (n° 29)

Une solution du composé n° 28 (2,72 g, 0,928 mmol) et de benzylamine (3,9 mL, 35,2 mmol) dans le tétrahydrofurane est agitée à température ambiante, pendant 16 heures. Le mélange réactionnel est dilué avec de l'acétate d'éthyle, lavé avec de l'acide chlorhydrique 1 M, de l'eau, séché sur sulfate de sodium, filtré puis concentré sous vide. La purification du résidu par chromatographie sur colonne de gel de silice [cyclohexane/acétone 4/5 (v/v)] donne 2,21 g du composé n° 29.
CCM sur gel de silice, cyclohexane/acétone [4/5 (v/v)] : Rf = 0,42

### PREPARATION 27

### Préparation du (2,3,4,6-Tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-6-O-acétyl-2-azido-2-désoxy-3-O-méthyl-D-glucopyranose trichloroacétimidate (n° 30)

Du trichloroacétonitrile (54,6 µL, 541 µmol) et du carbonate de césium (56,4 mg, 173 µmol) sont ajoutés à une solution du composé n° 29 (0,313 g, 108 µmol) dans le dichlorométhane (3 mL). Après agitation pendant 1 heure 30, le mélange est filtré puis concentré. Le résidu est purifié par chromatographie sur colonne de gel de silice en utilisant pour l'élution un mélange de cyclohexane/acétate d'éthyle/éthanol [2/0,5/0,5 (v/v/v)], contenant 0,1% de triéthylamine pour donner 245 mg du composé n° 30.
CCM sur gel de silice, cyclohexane/acétate d'éthyle/éthanol [5/1,5/1,5 (v/v/v)] ; Rf = 0,41

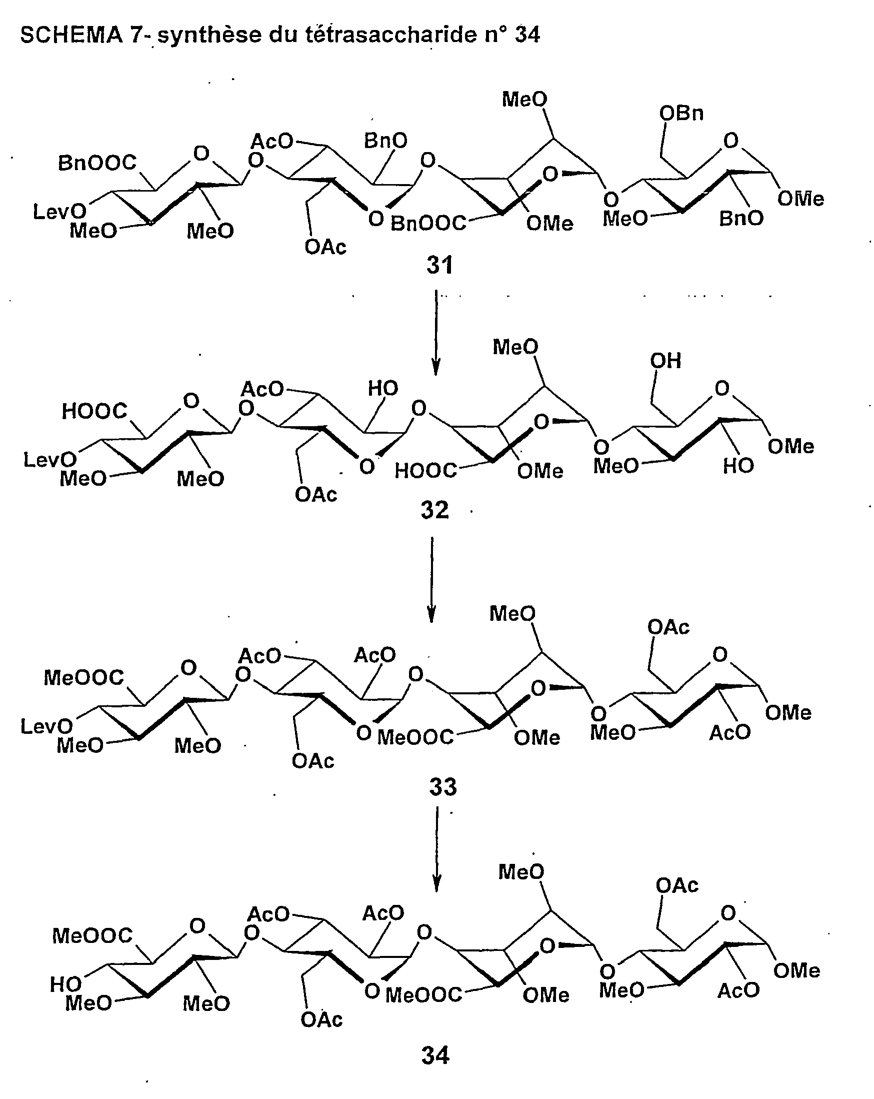

### PREPARATION 28

### Préparation du méthyl (acide 4-O-lévulinyl-2,3-di-O-méthyl-β-D-glucopyranosyluronique)-(1→4)-(3,6-di-O-acétyl-α-D-glucopyranosyl)-(1→4)-(acide 2,3-di-O-méthyl-α-L-idopyranosyluronic)-(1→4)-3-O-méthyl-α-D-glucopyranoside (n°32)

Une solution du composé n° 31 (4,50 g, 3,02 mmol), préparé par analogie avec la préparation 31 décrite dans la demande de brevet publiée sous le n° WO 99/36443, dans 72 mL d'un mélange acétyle d'éthyle/tert-butanol [(1/1 (v/v)] est traitée sous pression d'hydrogène (4 bar) en présence de palladium sur charbon 10% (9,0 g) pendant 6 heures. Après filtration et concentration, le composé n° 32 obtenu est directement engagé dans l'étape suivante sans purification.

### PREPARATION 29

### Préparation du méthyl(méthyl 4-O-lévulinyl-2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,6-di-O-acétyl-3-O-méthyl-α-D-glucopyranoside (n° 33)

A une solution du composé n° 32 (1,09 g, 1,13 mmol) dans du N,N-diméthylformamide anhydre (15 mL) on ajoute, à 0 °C, de l'hydrogénocarbonate de potassium (1,13 g) puis de l'iodure de méthyle (1,4 mL). Après 16 heures d'agitation à température ambiante, le milieu réactionnel est refroidi à 0 °C. On ajoute alors successivement de la diméthylaminopyridine (44 mg) puis de l'anhydride acétique (2,4 mL). Le mélange est agité pendant 16 heures. Après neutralisation de l'excès d'anhydride acétique, on dilue avec de l'acétate d'éthyle. La phase organique est lavée successivement avec une solution d'hydrogénosulfate de potassium à 10% et de l'eau puis avec une solution d'hydrogénocarbonate de sodium sarurée et de l'eau. La phase organique est ensuite séchée sur sulfate de sodium anhydre, filtrée puis évaporée à sec. Le résidu obtenu est remis à acétyler dans les conditions classiques (anhydride acétique, diméthylaminopyridine, triéthylamine dans le dichlorométhane). Après traitement, le résidu est purifié par chromatographie sur colonne de gel de silice [cyclohexane/acétate d'éthyle/éthanol 12/2,5/2,5 (v/v/v)] pour donner 0,910 g du composé n° 33.
CCM sur gel de silice, toluène/acétone [2/1 (v/v)] : Rf = 0,49

### PREPARATION 30

### Préparation du méthy(méthyl 2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,6-di-O-acétyl-3-O-méthyl-α-D-glucopyranoside (n° 34)

Le composé n° 33 (0,884 g, 0,793 mmol) est dissous dans 160 mL d'un mélange toluène/éthanol [1/1 (v/v)]. On ajoute de l'acétate d'hydrazine (0,365 mg). Après 5 heures d'agitation à température ambiante, le milieu réactionnel est concentré à sec. Le résidu est dissous dans le dichlorométhane. La phase organique est lavée successivement avec une solution d'hydrogénocarbonate dé sodium-à-2% et de l'eau, puis séchée sur sulfate de sodium anhydre, filtrée et évaporée à sec. Après chromatographie sur colonne de gel de silice [toluène/acétone 5/3 (v/v)], on obtient 0,696 g du composé n° 34.
CCM sur gel de silice, toluène/acétone [2/1 (v/v)] : Rf = 0,37

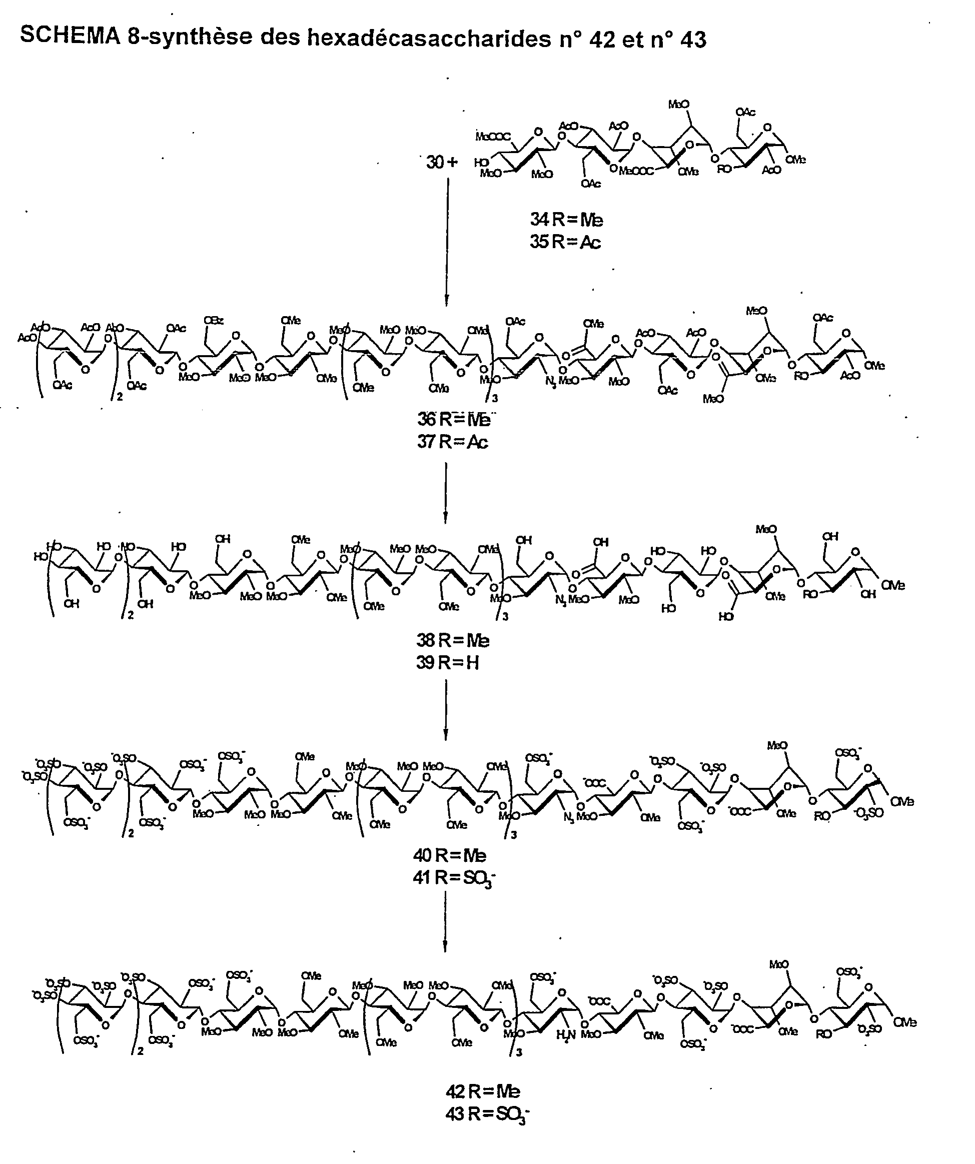

### PREPARATION 31

### Préparation du méthyl(2,3,4,6-tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-acétyl-2-azido-2-désoxy-3-O-méthyl-α-D-glucopyranosyl)-(1→4)-(méthyl 2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,6-di-O-acétyl-3-O-méthyl-α-D-glucopyranoside (n° 36)

On dissout le composé imidate n° 30 (170 mg, 56 µmol) obtenu à la PREPARATION 27, et le composé n° 34 (114 mg, 112 µmol) obtenu à la PREPARATION 30 dans 2,5 mL d'un mélange dichlorométhane/diéthyléther [1/2 (v/v)]. Après addition de tamis moléculaire 4Å en poudre, le mélange est refroidi à - 20 °C et on ajoute une solution de trifluorométhanesulfonate de triméthylsilyle . 0,1 M dans le dichlorométhane (84 µL). Après 40 minutes, le mélange est neutralisé par addition d'hydrogénocarbonate de sodium solide. Après filtration et concentration, le résidu est purifié par chromatographie sur gel Sephadex^{®} LH60, suivi d'une chromatographie sur colonne de gel de silice [éther diéthylique/éthanol (17/2 v/v)] pour donner 123 mg du composé n° 36.

Masse : méthode "ESI", mode positif : masse chimique = 3890,87; masse expérimentale : 3890,46 ± 0,68 u.m.a.
CCM sur gel de silice, éther diéthylique/éthanol [17/2 (v/v)] : Rf = 0,40

### PREPARATION 32

### Préparation du méthyl (2,3,4,6-tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-O-benzoyl-2,3-di-O-méthyl-α-D-glucopyranosyl)-(1→4)-(2,3,6-tri-O-methyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-acétyl-2-azido-2-désoxy-3-O-méthyl-α-D-glucopyranosyl)-(1→4)-(méthyl 2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,3,6-tri-O-acétyl-α-D-glucopyranoside (n° 37)

On dissout le composé imidate n° 30 obtenu selon la PREPARATION 27 (95 mg, 0,031 mmol) et le composé n° 35 (65,4 mg, 0,062 mmol), obtenu dans la préparation 42 de la demande de brevet publiée sous le numéro WO 02/24754, dans 1,5 mL d'un mélange dichlorométhane/diéthyléther [1/2 (v/v)]. Après addition de tamis moléculaire 4Å en poudre; le mélange est refroidi à - 20°C et on ajoute une solution de trifluorométhanesulfonate de triméthylsilyle 0,1 M (47 µL) dans le dichlorométhane (47 µL). Après 40 minutes, le mélange est neutralisé par addition d'hydrogénocarbonate de sodium solide. Après filtration et concentration, le résidu est purifié par chromatographie sur gel Sephadex^{®} LH60 (2 chromatographies ont été réalisées) pour donner 68 mg du composé 37.
Masse : méthode "ESI", mode positif : masse chimique = 3918,88 ; masse expérimentale : 3919,35 ± 0,71 u.m.a.
CCM sur gel de silice, cyclohexane/acétate d'éthyle/éthanol [3/1/1 (v/v/v)] : Rf = 0,53

### PREPARATION 33

### Préparation du méthyl (α-D-gluclopyranosyl)-(1→4)-(α-D-glucopyranosyl)-(1→4)-β-D-glucopyranosyl)-(1→4)-(2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-azido-2-désoxy-3-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)-(1→4)-3-O-méthyl-α-D-glucopyranoside (n° 38)

De l'eau oxygénée à 30% (3,9 mL) est ajoutée, à - 5°C, à une solution du composé n° 36 (111 mg) obtenu à la PREPARATION 31 dans le tétrahydrofurane (4,6 mL). Après 5 minutes d'agitation, on ajoute goutte à goutte une solution aqueuse d'hydroxyde de lithium 0,7 M (1,8 mL). Le mélange réctionnel est agité pendant 1 heure à - 5 °C, puis pendant 4 heures à 0 °C et finalement pendant 16 heures à température ambiante. On dépose le mélange réactionnel sur une colonne de Sephadex^{®} G-25 fine (5 x 100 cm) éluée par de l'eau. Les fractions contenant le composé attendu sont réunies, concentrées et déposées sur une colonne de résine Dowex^{®} AG 50 WX4 H⁺ (1,9 mL). On recueille le composé à 0 °C et on concentre pour obtenir 72,1 mg du composé n° 38.
CCM sur gel de silice, acétate d'éthyle/pyridine/acide acétique/eau [16/12/2,6/7 (v/v/v/v)] : Rf = 0,60.

### PREPARATION 34

### Préparation du méthyl (α-D-glucopyranosyl)-(1→4)-(α-D-glucopyranosyl)-(1→4)-β-D-glucopyranosyl)-(1→4)-(2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-azido-2-désoxy-3-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)- (1→4)-α-D-glucopyranoside (n° 39)

De l'eau oxygénée à 30% (2,2 mL) est ajoutée, à - 5 °C, à une solution du composé n° 37 (60 mg) dans le tétrahydrofurane (5,5 mL). Après 5 minutes d'agitation, on ajoute goutte à goutte une solution aqueuse d'hydroxide de lithium 0,7 M (1 mL). Le mélange réactionnel est agité pendant 1 heure à - 5 °C, puis pendant 4 heures à 0 °C et finalement pendant 16 heures à température ambiante. On neutralise avec une solution d'acide chlorhydrique 1 M. On dépose la solution sur une colonne de Sephadex^{®} G-25 fine (5 x 100 cm) éluée par de l'eau. Les fractions contenant le composé attendu sont réunies, concentrées et déposées sur une colonne de résine Dowex^{®} AG 50 WX4 H⁺. On recueille le composé à 0 °C et on concentre pour obtenir 37,5 mg du composé n° 39.
CCM sur gel de silice, acétate d'éthyle/pyridine/acide acétique/eau [16/12/2,6/7 (v/v/v/v)] : Rf = 0,36

### PREPARATION 35

### Préparation du méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-azido-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-3-O-méthyl-2,6-di-O-sulfonato-α-D-glucopyranoside, sel de sodium (n° 40)

Juste avant de l'utiliser, le composé n° 38 obtenu à la PREPARATION 33, est codistillé avec du N,N-diméthylformamide (3 x 2 mL). A une solution du composé n° 38 (70,3 mg, 22,8 µmol) dans le N,N-diméthylformamide (2 mL), on ajoute le complexe trioxyde de sulfure-triéthylamine (351 mg). Le mélange est agité pendant 16 heures à 55°C à l'abri de la lumière. Le mélangé, refroidi à 0 °C, est ajouté goutte à goutte à une solution d'hydrogénocarbonate de sodium dans l'eau. On agite pendant 16 heures à température ambiante et on concentre à sec. Le résidu, dissous dans l'eau, est déposé sur une colonne de Sephadex^{®} G-25 fine éluée par du chlorure de sodium 0,2 M. On concentre les fractions contenant le produit et on dessale en utilisant la même colonne éluée par l'eau. Après lyophilisation, on obtient 103 mg du composé n° 40.
Masse : méthode "ESI", mode négatif : masse chimique = 4864,82 ; masse expérimentale : 4862,90 ± 0,21 u.m.a.

### PREPARATION 36

### Préparation du méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-(α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-azido-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (n° 41)

Le composé n° 39 (33 mg, 0,017 mmol) obtenu à la PREPARATION 34 est transformé en composé n°41 selon le mode opératoire décrit à la PREPARATION 35. Après concentration, on obtient 40 mg du composé n° 41.
Masse : méthode "ESI", mode négatif : masse chimique = 4952,83 ; masse expérimentale : 4950,19 ± 0,55 u.m.a.

### PREPARATION 37

### Préparation du méthyl (2,3,4,6-tétra-O-sulfonafo-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-α-D-glucopyranosyl)-(1→4)]₃-(2-amino-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-3-O-méthyl-2,6-di-O-sulfonato-α-D-glucopyranoside, sel de sodium (n° 42)

Une solution du composé n° 40 (93,8 mg) obtenu à la PREPARATION 35 dans un mélange tert-butanol (1,2 mL) et eau (1,8 mL), est traitée sous pression d'hydrogène (5 bar) en présence de palladium sur charbon 10% (28 mg) à 40 °C pendant 4 heures. Après filtration (filtre Millipore^{®} LSWP 5 µm), la solution est concentrée à sec pour donner 93 mg du composé n° 42.

### PREPARATION 38

### Préparation du méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-suifonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-amino-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (n° 43)

Le composé n° 41 (40,8 mg) obtenu à la PREPARATION 36, est transformé en composé n° 43 selon le mode opératoire décrit à la PREPARATION 37. Après concentration, on obtient 42,3 mg du composé n° 43. Masse méthode "ESI", mode négatif : masse chimique = 4926,84 ; masse expérimentale : 4924,07 ±0,36 u.m.a.

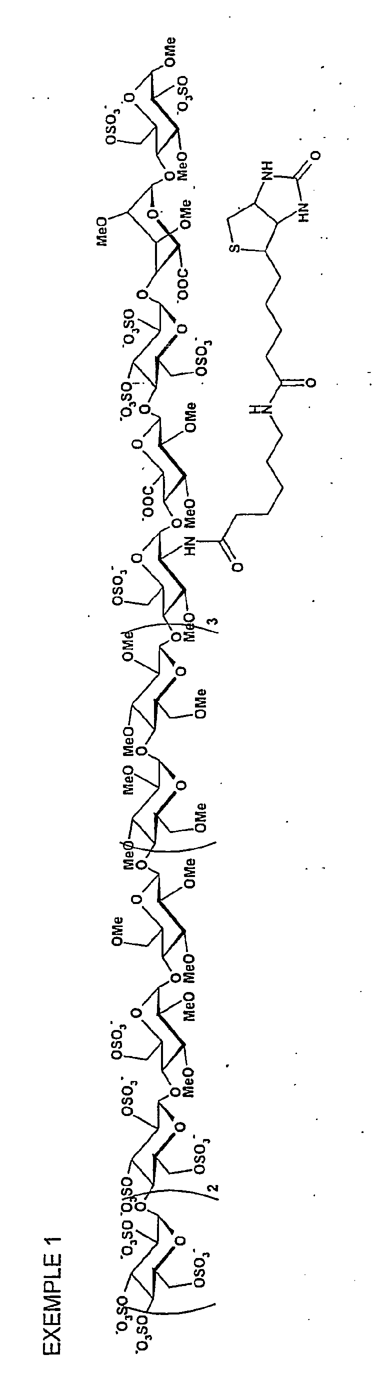

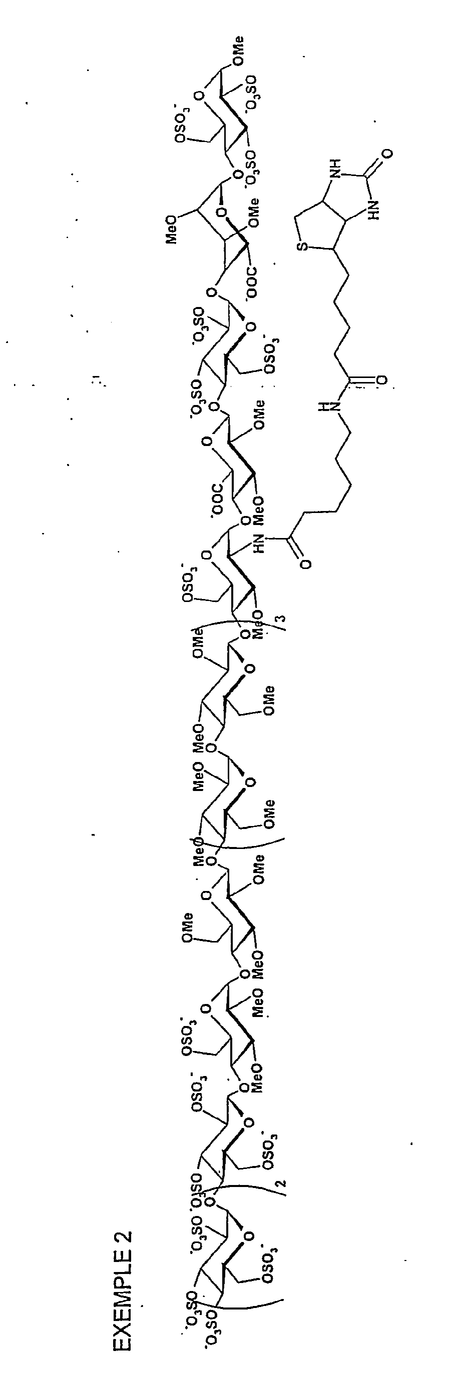

### EXEMPLE 1

### Préparation du méthyl (2,3,4,6-tétra-O-sulfonato-α-D-glucopyranosy)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-[N-(6-biotinamidohexanoyl)]-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-3-O-méthyl-2,6-di-O-sulfonato-α-D-glucopyranoside, sel de sodium

Le composé n° 42 (20 mg, 4,13 µmol) obtenu selon la PREPARATION 37, est dissous dans une solution aqueuse d'hydrogénocarbonate de sodium à 0,5% (1,7 mL). On y ajoute goutte à goutte une solution de 6-(biotinamido) hexanoate de sulfosuccinimide (23 mg, 41,3 µmol) dans une solution d'hydrogénocarbonate de sodium à 0,5% (100 µL). Après 16 heures d'agitation à température ambiante, on ajoute une solution aqueuse d'hydroxyde de sodium 1 M et on agite pendant 1 heure. On dépose le mélange réactionnel sur une colonne de Sephadex^{®} G-25 fine (5 x 100 cm) éluée par une solution aqueuse du chlorure de sodium 0,2 M. On concentre les fractions contenant le produit et on dessale en utilisant la même colonne éluée par l'eau. Après lyophilisation, on obtient 21,2 mg du composé de l'EXEMPLE 1.
Masse : méthode "ESI"; mode négatif : masse monoisotopique = 5174,38 ; masse chimique =5178,28 ; masse expérimentale : 5177,69 ± 0,52 u.m.a.

### EXEMPLE 2

### Préparation du méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-[N-(6-biotinamidohexanoyl)]-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

Le composé n° 43 (19,1 mg) obtenu selon la PREPARATION 38, est transformé en composé n°45 selon le mode opératoire décrit dans l'EXEMPLE 1. Après lyophilisation, on obtient 18,1 mg du composé de l'EXEMPLE 2. Masse : méthode "ESI", mode négatif : masse monoisotopique = 5262,31 ; masse chimique = 5266,30 ; masse expérimentale : 5263,93 ± 0,38 u.m.a.

## Revendications

1. Hexadécasaccharides biotinylés de formule générale I : 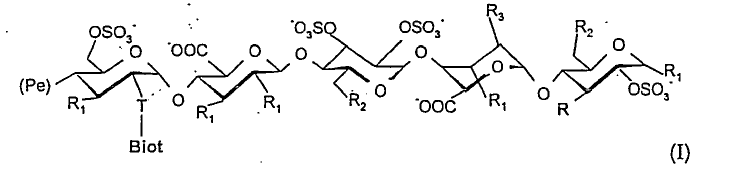 dans laquelle :
- T représente un enchaînement T₁ ou T₂ de formules suivantes: 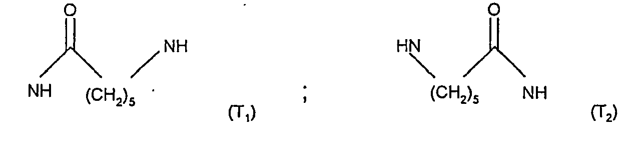
- Biot représente le groupe : 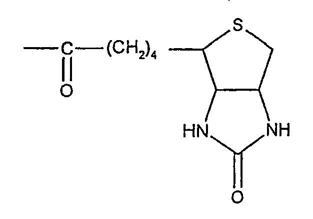
- R représente un radical (C₁-C₆)alcoxy, notamment un radical méthoxy ou un radical -OSO₃⁻,
- R₁ représente un radical (C₁-C₆)alcoxy, notamment un radical méthoxy ou un radical -OSO₃⁻,
- R₂ représente un radical (C₁-C₆)alcoxy ou un radical -OSO₃⁻,
- R₃ représente un radical (C₁-C₆)alcoxy, notamment un radical méthoxy ou un radical -OSO₃⁻, ou bien R₃ constitue un point -O-CH₂-, le groupe -CH₂- étant lié à l'atome de carbone porteur de la fonction carboxylique sur le même cycle,
- Pe représente un enchaînement saccharidique de formule suivante : 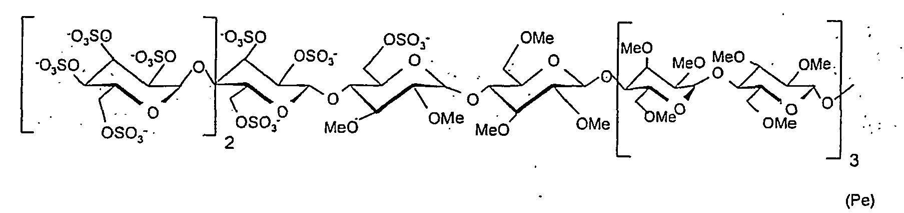
ainsi que leurs sels pharmaceutiquement acceptables.

2. Hexadécasaccharides biotinylés selon la revendication 1 de formule générale I dans laquelle :
- R représente un radical méthoxy ou un radical -OSO₃⁻,
- R₁ représente un radical méthoxy,
- R₂ représente un radical -OSO₃⁻,
- R₃ représente un radical méthoxy,

3. Hexadécasaccharides biotinylés selon l'une quelconque des revendications 1 ou 2, choisis parmi :
• Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-[N-(6-biotinamido hexanoyl)]-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-3-O-méthyl-2,6-di-O-sulfonato-α-D-glucopyranoside, sel de sodium
• Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-[N-(6-biotinamido hexanoyl)]-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

4. Compositions pharmaceutiques contenant, en tant que principe actif, un hexadécsaccharide selon l'une quelconque des revendications 1 à 3 éventuellement en association avec un ou plusieurs excipients inertes et appropriés.

5. Utilisation des compositions pharmaceutiques selon la revendication 4, pour la fabrication d'un médicament utile pour le traitement des pathologies consécutives à une modification de l'homéostasie du système de la coagulation apparaissant lors des troubles du système cardio-vasculaire et cérébro-vasculaire comme les troubles thromboemboliques associés à l'arthérosclérose et au diabète tels que l'angine instable, l'attaque cérébrale, la resténose après angioplastie, l'endartérectomie, la pose de prothèses endovasculaires, ou les troubles thromboemboliques associés à la rethrombose après thrombolyse, à l'infarctus, à la démence d'origine ischémique, aux maladies artérielles périphériques, à l'hémodialyse, aux fibrillations auriculaires, lors de l'utilisation de prothèses vasculaires de pontages aorto-coronariens, utile pour le traitement ou la prévention de pathologies thromboemboliques d'origine veineuse telles les embolies pulmonaires, et utile pour le traitement ou la prévention des complications thrombotiques observés à la suite d'opérations chirurgicales, de développements tumoraux ou de dérèglements de la coagulation, induits par des activateurs bactériens, viraux, ou enzymatiques.

6. Utilisation d'hexadécasaccharides biotinylés selon l'une quelconque des revendications 1 à 3 pour la fabrication d'un médicament utile pour recouvrir des prothèses.

7. Utilisation d'hexadécasaccharides biotinylés selon l'une quelconque des revendications 1 à 3 pour la fabrication d'un médicament utile comme adjuvant lors d'endartérectomie réalisée avec des ballonnets poreux.

8. Utilisation d'avidine ou de streptavidine pour la préparation de médicaments destinés à neutraliser l'activité antithrombotique des hexadécasaccharides biotinylés selon l'une quelconque des revendications 1 à 3.

## Claims

1. Biotinylated hexadecasaccharides of general formula I: 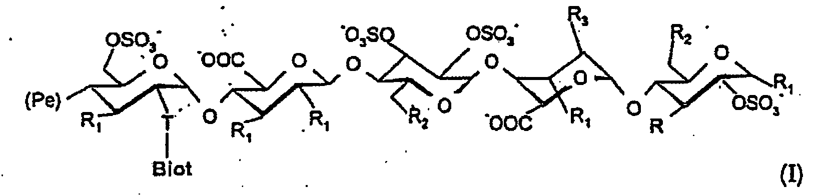 in which:
- T represents a T₁ or T₂ linkage of following formulae: 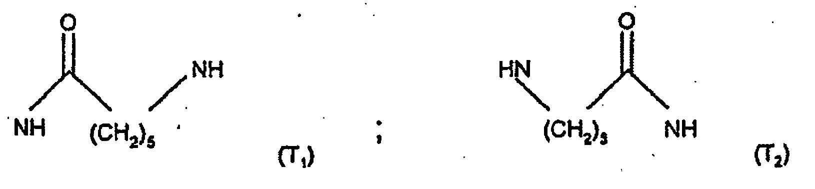
- Biot represents the group: 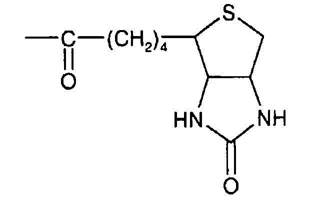
- R represents a (C₁-C₆)alkoxy radical, in particular a methoxy radical, or an -OSO₃⁻ radical,
- R₁ represents a (C₁-C₆) alkoxy radical, in particular a methoxy radical, or an -OSO₃⁻ radical,
- R₂ represents a (C₁-C₆) alkoxy radical or an -OSO₃⁻ radical,
- R₃ represents a (C₁-C₆)alkoxy radical, in particular a methoxy radical, or an -OSO₃⁻ radical, or else R₃ constitutes an -O-CH₂-bridge, the -CH₂- group being bonded to the carbon atom carrying the carboxyl functional group on the same ring,
- Pe represents a saccharide linkage of following formula: 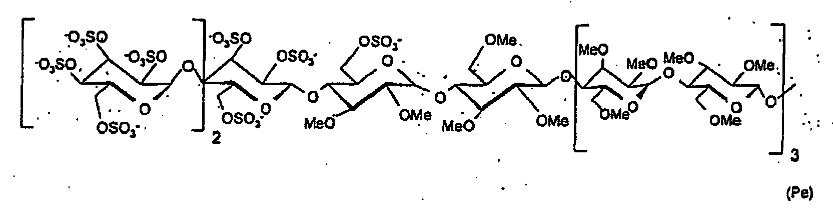
and their pharmaceutically acceptable salts.

2. Biotinylated hexadecasaccharides according to Claim 1 of general formula I in which:
- R represents a methoxy radical or an -OSO₃⁻ radical,
- R₁ represents a methoxy radical,
- R₂ represents an -OSO₃⁻ radical,
- R₃ represents a methoxy radical.

3. Biotinylated hexadecasaccharides according to either one of Claims 1 and 2, chosen from:
• Methyl (2,3,4,6-tetra-*O*-sulphonato-α-D-glucopyranosyl)-(1→4)-(2,3,6-tri-*O*-sulphonato-α-D-glucopyranosyl)-(1→4)-(2,3,6-tri-*O-*sulphonato-β-D-glucopyranosyl)-(1→4)-(2,3-di-*O*-methyl-6-*O*-sulphonato-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O-*methyl-β-D-glucopyranosyl)-(1→4)]₃-(2-[N-(6-biotinamidohexanoyl)]-2-deoxy-3-*O*-methyl-6-*O-*sulphonato-α-D-glucopyranosyl)-(1→4)-(2,3-di-*O*-methyl-β-D-glucopyranosyluronic acid)-(1→4)-(2,3,6-tri-*O*-sulphonato-α-D-glucopyranosyl)-(1→4)-(2,3-di-*O*-methyl-α-L-idopyranosyluronic acid)-(1→4)-3-*O*-methyl-2,6-di-*O*-sulphonato-α-D-glucopyranoside, sodium salt,
• Methyl (2,3,4,6-tetra-*O*-sulphonato-α-D-glucopyranosyl)-(1→4)-(2,3,6-tri-*O*-sulphonato-α-D-glucopyranosyl)-(1→4)-(2,3,6-tri-*O-*sulphonato-β-D-glucopyranosyl)-(1→4)-(2,3-di-*O*-methyl-6-*O*-sulphonato-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O*-methyl-β-D-glucopyranosyl)-(1→4)]₃-(2-[N-(6-biotinamidohexanoyl)]-2-deoxy-3-*O*-methyl-6-*O-*sulphonato-α-D-glucopyranosyl)-(1→4)-(2,3-di-*O*-methyl-β-D-glucopyranosyluronic acid)-(1→4)-(2,3,6-tri-*O*-sulphonato-α-D-glucopyranosyl)-(1→4)-(2,3-di-*O*-methyl-α-L-idopyranosyluronic acid)-(1→4)-2,3,6-tri-*O-*sulphonato-α-D-glucopyranoside, sodium salt.

4. Pharmaceutical compositions comprising, as active principle, a hexadecasaccharide according to any one of Claims 1 to 3, optionally in combination with one or more inert and appropriate excipients.

5. Use of the pharmaceutical compositions according to Claim 4, in the manufacture of a medicament of use in the treatment of pathologies resulting from a modification of the homoeostasis of the coagulation system appearing during disorders of the cardiovascular and cerebrovascular system, such as thromboembolic disorders associated with atherosclerosis and diabetes, for example unstable angina, apoplexy, postangioplasty restenosis, endarterectomy or the insertion of endovascular prostheses, or thromboembolic disorders associated with post-thrombolyses rethrombosis, with infarction, with dementia of ischaemic origin, with peripheral arterial diseases, with haemodialysis or with auricular fibrillations, during the use of vascular prostheses for aortocoronary bypasses, of use in the treatment or prevention of thromboembolic pathologies of venous origin, such as pulmonary embolisms, and of use in the treatment or prevention of thrombotic complications observed following surgical operations, the growth of tumours or disturbances to coagulation induced by bacterial, viral or enzymatic activators.

6. Use of biotinylated hexadecasaccharides according to any one of Claims 1 to 3 in the manufacture of a medicament of use in covering prostheses.

7. Use of biotinylated hexadecasaccharides according to any one of Claims 1 to 3 in the manufacture of a medicament of use as adjuvant during endarterectomy carried out with porous balloons.

8. Use of avidin or of streptavidin in the preparation of medicaments intended to neutralize the antithrombotic activity of the biotinylated hexadecasaccharides according to any one of Claims 1 to 3.

## Patentansprüche

1. Biotinylierte Hexadecasaccharide der allgemeinen Formel I: 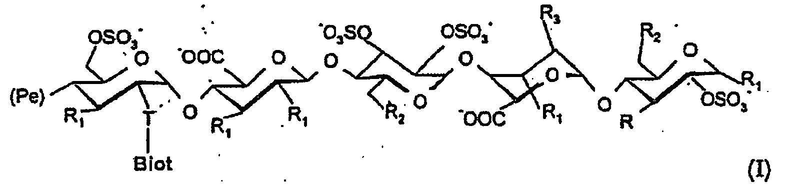
- T für eine Kette T₁ oder T₂ der folgenden Formeln steht: 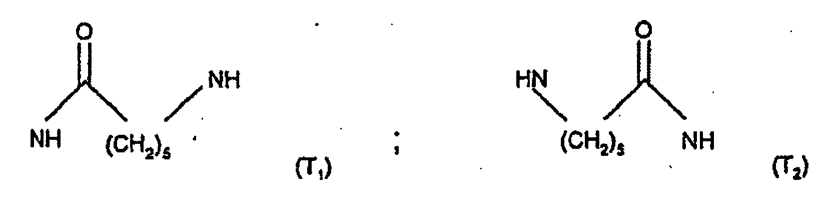
- Biot für die Gruppe: 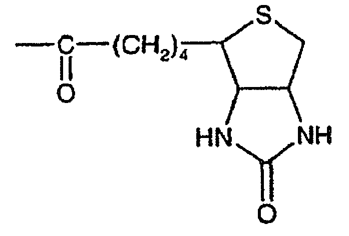 steht,
- R für einen (C₁-C₆)-Alkoxyrest, insbesondere einen Methoxyrest oder einen OSO₃⁻-Rest steht,
- R₁ für einen (C₁-C₆)-Alkoxyrest, insbesondere einen Methoxyrest oder einen OSO₃⁻-Rest steht,
- R₂ für einen (C₁-C₆)-Alkoxyrest oder einen OSO₃⁻-Rest steht,
- R₃ für einen (C₁-C₆)-Alkoxyrest, insbesondere einen Methoxyrest oder einen OSO₃⁻-Rest steht oder auch eine -O-CH₂-Brücke darstellt, wobei die -CH₂-Gruppe an das die Carboxylfunktion desselben Rings tragende Kohlenstoffatom gebunden ist;
- Pe für eine Saccharidkette der folgenden Formel steht: 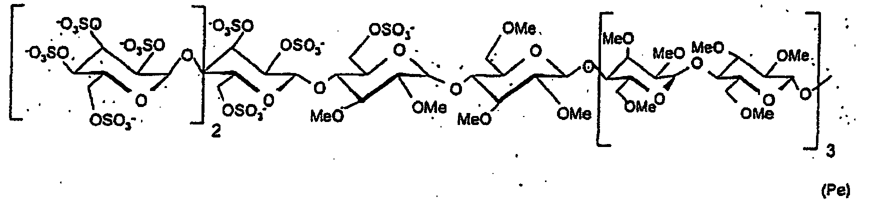
und pharmazeutisch unbedenkliche Salze davon.

2. Biotinylierte Hexadecasaccharide nach Anspruch 1 der allgemeinen Formel I, worin:
- R für einen Methoxyrest oder einen OSO₃⁻-Rest steht,
- R₁ für einen Methoxyrest steht,
- R₂ für einen OSO₃⁻-Rest steht,
- R₃ für einen Methoxyrest steht.

3. Hexadecasaccharide nach Anspruch 1 oder 2, ausgewählt unter:
• Methyl-(2,3,4,6-tetra-*O*-sulfonato-α-D-glucopyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(2,3-di-*O*-methyl-6-O-sulfonato-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O*-methyl-β-D-glucopyranosyl)-(1→4)]₃-(2-[N-(6-biotinamidohexanoyl)]-2-desoxy-3-O-methyl-6-*O-*sulfonato-α-D-glucopyranosyl)-(1→4)-(2,3-di-*O-*methyl-β-D-glucopyranosyluronsäure)-(1→4)-(2,3,6-tri-*O*-sulfonato-α-D-glucopyranosyl)-(1→4)-(2,3-di-*O*-methyl-α-L-idopyranosyl-uronsäure)-(1→4)-3-O-methyl-2,6-di-*O*-sulfonato-α-D-glucopyranosid-Natriumsalz,
• Methyl-(2,3,4,6-tetra-*O*-sulfonato-α-D-glucopyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(2,3-di-O-methyl-6-O-sulfonato-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O-*(2,3,6-tri-*O-*methyl-β-D-glucopyranosyl)-(1→4)]3-(2-[N-(6-biotinamidohexanoyl)]-2-desoxy-3-O-methyl-6-*O*-sulfonato-α-D-glucopyranosyl)-(1→4)-(2,3-di-*O*-methyl-β-D-glucopyranosyluronsäure)-(1→4)-(2,3,6-tri-*O-*sulfonato-α-D-glucopyranosyl)-(1→4)-(2,3-di-*O-*methyl-α-L-idopyranosyluronsäure)-(1→4)-2,3,6-tri-*O*-sulfonato-α-D-glucopyranosid-Natriumsalz.

4. Pharmazeutische Zusammensetzungen, enthaltend als Wirkstoff ein Hexadecasaccharid nach einem der Ansprüche 1 bis 3, gegebenenfalls in Kombination mit einem oder mehreren inerten und geeigneten Trägerstoffen.

5. Verwendung der pharmazeutischen Zusammensetzungen nach Anspruch 4 zur Herstellung eines Arzneimittels zur Behandlung von Folgepathologien einer Modifizierung der Homöostase des Koagulationssystems, die bei Störungen des kardiovaskulären oder zerebrovaskulären Systems wie mit Arteriosklerose und Diabetes assoziierten thromboembolischen Störungen, wie instabiler Angina, Hirnschlag, Restenose nach Angioplastie, Endarteriektomie oder dem Einsetzen von endovaskulären Prothesen, oder mit Rethrombose nach Thrombolyse, Infarkt, Demenz ischämischen Ursprungs, peripheren arteriellen Erkrankungen, Hämodialyse oder Vorhofflimmern assoziierten thromboembolischen Störungen oder bei der Verwendung von Gefäßprothesen bei aortokoronaren Bypässen auftreten, bei der Behandlung oder Prävention von thromboembolischen Erkrankungen venösen Ursprungs wie Lungenembolien, zur Behandlung oder Prävention von thrombotischen Komplikationen nach chirurgischen Operationen, Tumorwachstum oder durch bakterielle, virale oder enzymatische Aktivatoren induzierten Koagulationsstörungen.

6. Verwendung biotinylierten Hexadecasacchariden nach einem der Ansprüche 1 bis 3 zur Herstellung eines Arzneimittels zur Beschichtung von Prothesen.

7. Verwendung biotinylierten Hexadecasacchariden nach einem der Ansprüche 1 bis 3 zur Herstellung eines Arzneimittels als Adjuvans bei der Endarteriektomie mit porösen Ballons.

8. Verwendung von Avidin oder Streptavidin zur Herstellung von Arzneimitteln zur Neutralisation der antithrombotischen Aktivität der biotinylierten Hexadecasacchariden nach einem der Ansprüche 1 bis 3.
